(19) Europäisches Patentamt
European Patent Office
Office européen des brevets

(11) **EP 2 372 359 B1**

(12) **EUROPEAN PATENT SPECIFICATION**

(45) Date of publication and mention
of the grant of the patent:
**06.01.2016 Bulletin 2016/01**

(51) Int Cl.:
**G01N 33/22** *(2006.01)*          **G01N 27/18** *(2006.01)*
**G01N 25/18** *(2006.01)*          **G01K 17/04** *(2006.01)*
**G01K 19/00** *(2006.01)*          *G01N 33/00* *(2006.01)*

(21) Application number: **11159686.2**

(22) Date of filing: **24.03.2011**

(54) **Calorific value calculation formula creation system, method of creating calorific value calculation formula, calorific value measurement system and method of measuring calorific value**

Formelerzeugungssystem zur Heizwertberechnung, Verfahren zur Erzeugung der Formel zur Heizwertberechnung, Messsystem des Heizwerts und Verfahren zum Messen des Heizwerts

Système de création de formule de calcul de la valeur calorifique, procédé de création de formule de calcul de la valeur calorifique, système de mesure de la valeur calorifique et procédé de mesure de la valeur calorifique

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2010 JP 2010075097**

(43) Date of publication of application:
**05.10.2011 Bulletin 2011/40**

(73) Proprietor: **Azbil Corporation**
**Chiyoda-ku**
**Tokyo 100-6419 (JP)**

(72) Inventors:
• **Mutou, Hiroyuki**
**Tokyo 100-6419 (JP)**
• **Ooishi, Yasuharu**
**Tokyo 100-6419 (JP)**
• **Seita, Misako**
**Tokyo 100-6419 (JP)**

(74) Representative: **Tomlinson, Kerry John**
**Dehns**
**St Bride's House**
**10 Salisbury Square**
**London**
**EC4Y 8JD (GB)**

(56) References cited:
**EP-A1- 1 193 488     EP-A1- 1 947 450**
**WO-A2-01/79830     US-A- 5 311 447**

• **A.J. SMOLA, B. SCHOLKOPF: "A Tutorial on Support Vector Regression", NEUROCOLT TECHNICAL REPORT (NC-TR-98-030), 1998, XP002638775,**

## Description

Technical Field

The present invention relates to a gas examination technique, and more particularly, to a calorific value calculation formula creation system, a method of creating a calorific value calculation formula, a calorific value measurement system, and a method of measuring a calorific value.

Related Art

In the related art, when the calorific value of a mixed gas is calculated, it is necessary to analyze the components of the mixed gas using, for example, an expensive gas chromatography device. In addition, a method has been proposed which measures the thermal conductivity of a mixed gas and the speed of sound in the mixed gas to calculate the percentage of components, such as methane ($CH_4$), propane ($C_3H_8$), nitrogen ($N_2$), and carbon dioxide ($CO_2$), included in the mixed gas, thereby calculating the calorific value of the mixed gas (for example, see JP-T-2004-514138).

However, in the method disclosed in JP-T-2004-514138, an expensive sound speed sensor for measuring the speed of sound is required in addition to a sensor for measuring thermal conductivity.

EP 1947450 discloses an apparatus comprising: a microheater which is provided in an ambient gas; heater temperature detecting means for obtaining a temperature Th of the microheater; a temperature sensor which measures an ambient temperature To of the microheater; a power supply which energizes the microheater to generate heat; heat radiation coefficient computing means for calculating a heat radiation coefficient C from the microheater as [C=Ph/(Th-To)] in accordance with an energization power Ph; and thermal conductivity computing means for obtaining a thermal conductivity $\lambda_{(T)}$ of the ambient gas at the measurement temperature T from the heat radiation coefficient C calculated by the heat radiation coefficient computing means based on a proportional relation [C=K·$\lambda_{(T)}$].

The apparatus obtains a thermal conductivity $\lambda_{(T)}$ of an ambient gas at each of different heater temperatures by using the thermal conductivity measuring apparatus; and analyzes composition ratios of the ambient gas based on a simultaneous equation of the thermal conductivity $\lambda_{(T)}$ at each of the heater temperatures. Specifically, the analyzing obtains the composition ratios by analyzing [n-1] thermal conductivities $\lambda_{(T1)}$, and $\lambda_{(T2)}$ to $\lambda_{(Tn-1)}$ acquired at heater temperatures Th(1), and Th(2) to Th(n-1) set on [n-1] stages on the assumption that the ambient gas is a mixed gas containing n types of gases and the thermal conductivity $\lambda_{(T)}$ of the mixed gas is obtained by adding thermal conductivities $\lambda1_{(T)}$, and $\lambda2_{(T)}$ to $\lambda n_{(T)}$ of the respective gases at ratios determined in accordance with composition ratios and coupling coefficients of the respective gases.

SUMMARY

According to a first aspect of the present invention, there is provided a calorific value calculation formula creating system comprising a container into which a gas mixture, including a plurality of gases and having a predetermined calorific value, may be injected; a temperature measurement element disposed in the container; a heater element which is disposed in the container and arranged to generate heat at a plurality of heating temperatures; a measurement module arranged to measure first values of electric signals generated from the temperature measurement element and second values of electric signals generated from the heater element at each of the heating temperatures, wherein the first values depend on temperatures of the temperature measurement element at the plurality of gases and the second values depend on temperatures of the heater element; and a formula creation module which creates a calorific value calculation formula based on the predetermined calorific values, the first values, and the second values of each of a plurality of gas mixtures, wherein the calorific value calculation formula is represented by: the first values and the second values, both of which are set as independent variables in the calorific value calculation formula; and a calorific value of a gas mixture that is set as a dependent variable in the calorific value calculation formula.

According to a second aspect of the present invention, there is provided a method of creating a calorific value calculation formula comprising: (a) providing a gas mixture, including a plurality of gases and having a predetermined calorific value, in a container; (b) measuring first values of electric signals generated from a temperature measurement element which is disposed in the container, wherein the first values depend on temperatures of the temperature measurement element at the plurality of gases; measuring second values of electric signals generated from a heater element at each of heating temperatures, wherein the heater element is disposed in the container and generates heat at the heating temperatures, and wherein the second values depend on temperatures of the heater element; (d) repeating steps (a) to (c) for a plurality of gas mixtures, each including a plurality of gases and having a predetermined calorific value; and (e) creating a calorific value calculation formula based on the predetermined calorific values, the first values, and the second values of each of the plurality of gas mixtures, wherein the calorific value calculation formula is represented by: the first values and the second values, both of which are set as independent variables in the calorific value calculation formula; and a calorific value of a gas mixture that is set as a dependent variable in the calorific value calculation formula.

BRIEF DESCRIPTION OF THE DRAWINGS

Fig. 1 is a perspective view illustrating a microchip according to a first embodiment of the invention;
Fig. 2 is a cross-sectional view illustrating the microchip according to the first embodiment of the invention taken along the line II-II of Fig. 1;
Fig. 3 is a circuit diagram illustrating a heater element according to the first embodiment of the invention;
Fig. 4 is a circuit diagram a heat-retaining element according to the first embodiment of the invention;
Fig. 5 is a graph illustrating the relationship between the radiation coefficient of gas and the temperature of the heater element according to the first embodiment of the invention;
Fig. 6 is a first schematic diagram of a calorific value calculation formula creation system according to the first embodiment of the invention;
Fig. 7 is a second schematic diagram of the calorific value calculation formula creation system according to the first embodiment of the invention;
Fig. 8 is a flowchart illustrating a method of creating a calorific value calculation formula according to the first embodiment of the invention;
Fig. 9 is a schematic diagram of a calorific value calculation formula creation system according to a second embodiment of the invention;
Fig. 10 is a schematic diagram of a calorific value calculation formula creation system according to a third embodiment of the invention;
Fig. 11 is a first graph showing errors from the calculated true values of calorific values of sample mixed gases according to Example 2 of the embodiment;
Fig. 12 is a second graph showing errors from the calculated true values of calorific values of sample mixed gases according to Example 2 of the embodiment; and
Fig. 13 is a third graph showing errors from the calculated true values of calorific values of sample mixed gases according to Example 2 of the embodiment.

DETAILED DESCRIPTION OF EXEMPLARY EMBODIMENTS

Hereinafter, exemplary embodiments of the invention will be described. In the following description of the drawings, the same or similar components are denoted by the same or similar reference numerals. However, the drawings are schematically illustrated. Therefore, for example, detailed dimensions need to be determined in the light of the following description. In addition, in the drawings, the same components may have different dimensions or ratios.

(First embodiment)

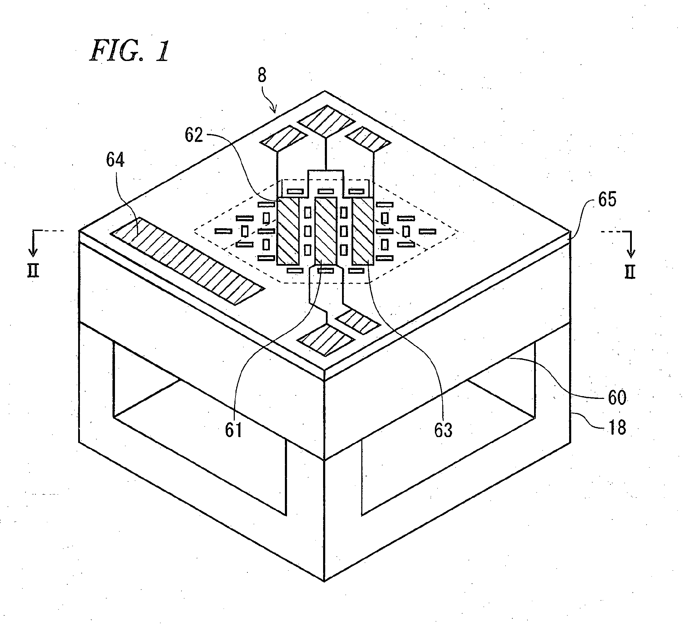

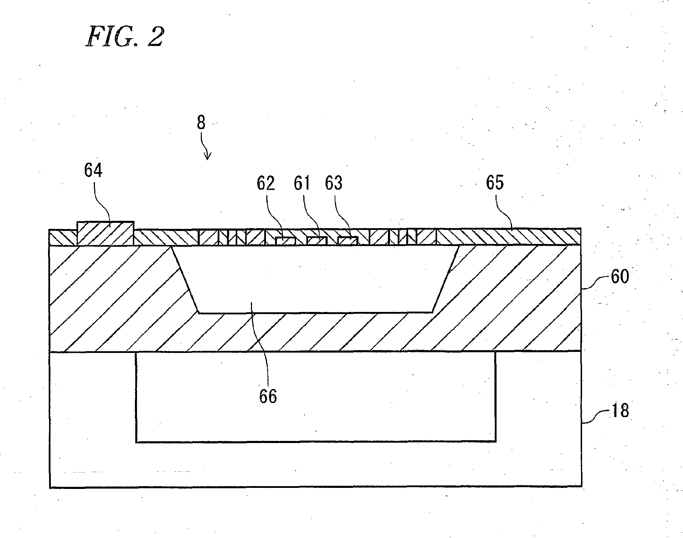

First, a microchip 8 used in a gas property value measurement system according to a first embodiment will be described with reference to Fig. 1, which is a perspective view, and Fig. 2, which is a cross-sectional view taken along the line II-II. The microchip 8 includes a substrate 60 having a cavity 66 provided therein and an insulating film 65 that is provided on the substrate 60 so as to cover the cavity 66. The thickness of the substrate 60 is, for example, 0.5 mm. In addition, the substrate 60 has, for example, a size of about 1.5 mm $\times$ 1.5 mm. A portion of the insulating film 65 covering the cavity 66 is a heat insulating diaphragm. The microchip 8 further includes a heater element 61 that is provided in the diaphragm portion of the insulating film 65, first and second temperature measurement elements 62 and 63 that are provided in the diaphragm portion of the insulating film 65 with the heater element 61 interposed therebetween, and a heat-retaining element 64 that is provided on the substrate 60.

The heater element 61 is arranged at the center of the diaphragm portion of the insulating film 65 covering the cavity 66. The heater element 61 is, for example, a resistor and is supplied with power to generate heat, thereby heating an atmospheric gas coming into contact with the heater element 61. The first temperature measurement element 62 and the second temperature measurement element 63 are, for example, electronic elements such as a passive element of a resistor or the like and are supplied with a weak voltage not enough to generate self-heating to output an electric signal depending on the gas temperature of an atmospheric gas. Not generating self-heating means that the temperatures of the first temperature measurement element 62 and the second temperature measurement element 63 are close to the atmospheric temperature. Hereinafter, an example will be described in which an output signal of the first temperature measurement element 62 is used, but the invention is not limited thereto. For example, an average value of an output signal of the first temperature measurement element 62 and an output signal of the second temperature measurement element 63 may be used as the output signal of the temperature measurement element.

The heat-retaining element 64 is, for example, a resistor and is supplied with power to generate heat, thereby constantly maintaining the temperature of the substrate 60 to, for example, 60°C. As a material for the substrate 60, silicon (Si) or the like can be used. As a material for the insulating film 65, silicon oxide ($SiO_2$) or the like can be used. The cavity 66 is formed by anisotropic etching or the like. In addition, platinum (Pt) or the like can be used as the materials for the heater element 61, the first temperature measurement element 62, the second temperature measurement element 63, and the heat-retaining element 64, and these can be formed using a lithography method. In addition, the heater element 61, the first temperature measurement element 62, and the second temperature measurement element 63 may be formed of the same members.

The microchip 8 is fixed to a container, such as a chamber filled up with an atmosphere gas, with a heat insulating member 18 which is provided on the bottom of the, microchip 8 interposed therebetween. When the microchip 8 is fixed to, for example, the chamber with the heat insulating member 18 interposed therebetween, the temperature of the microchip 8 is less likely to be affected by a variation in the temperature of the inner wall of the chamber. For example, the thermal conductivity of the heat insulating member 18 made of glass is equal to or less than 1.0 W/(m-K).

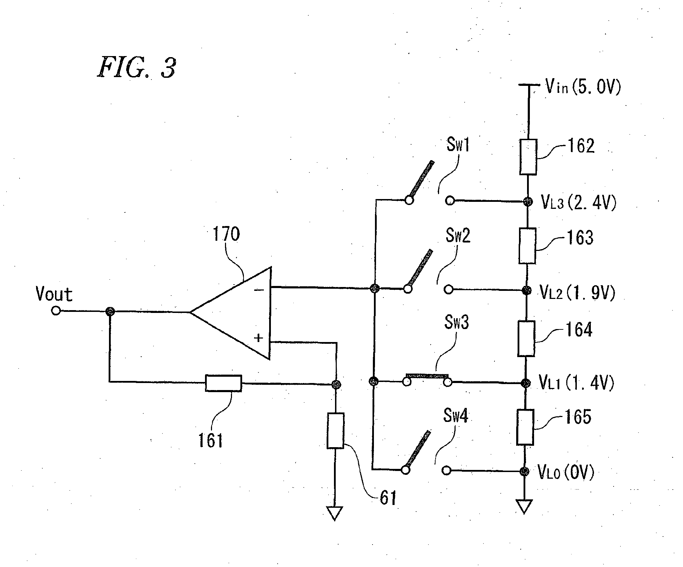

As shown in Fig. 3, the heater element 61 has one end that is electrically connected to, for example, a positive (+) input terminal of an operational amplifier 170 and the other end that is connected to the ground. A resistive element 161 is connected in parallel to the positive input terminal and an output terminal of the operational amplifier 170. A negative (-) input terminal of the operational amplifier 170 is electrically connected between a resistive element 162 and a resistive element 163 connected in series to each other, between the resistive element 163 and a resistive element 164 connected in series to each other, between the resistive element 164 and a resistive element 165 collected in series to each other, or to an earth terminal of the resistive element 165. The resistance values of the resistive elements 162 to 165 are appropriately determined, thereby for example, when a voltage Vin of 5.0 V is applied to one end of the resistive element 162, a voltage $V_{L3}$ of, for example, 2.4 V is generated between the resistive element 163 and the resistive element 162. In addition, a voltage $V_{L2}$ of, for example, 1.9 V is generated between the resistive element 164 and the resistive element 163 and a voltage $V_{L1}$ of, for example, 1.4 V is generated between the resistive element 165 and the resistive element 164.

A switch SW1 is provided between the positive input terminal of the operational amplifier and a point between the resistive element 162 and the resistive element 163, and a switch SW2 is provided between the positive input terminal of the operational amplifier and a point between the resistive element 163 and the resistive element 164. In addition, a switch SW3 is provided between the positive input terminal of the operational amplifier and a point between the resistive element 164 and the resistive element 165, and a switch SW4 is provided between the ground terminal of the resistive element 165 and the positive input terminal of the operational amplifier.

When a voltage $V_{L3}$ of 2.4 V is applied to the negative input terminal of the operational amplifier 170, only the switch SW1 is energized and the switches SW2, SW3, and SW4 are disconnected. When a voltage $V_{L2}$ of 1.9 V is applied to the negative input terminal of the operational amplifier 170, only the switch SW2 is energized and the switches SW1, SW3, and SW4 are disconnected. When a voltage $V_{L1}$ of 1.4 V is applied to the negative input terminal of the operational amplifier 170, only the switch SW3 is energized and the switches SW1, SW2, and SW4 are disconnected. When a voltage $V_{L0}$ of 0 V is applied to the negative input terminal of the operational amplifier 170, only the switch SW4 is energized and the switches SW1, SW2, and SW3 are disconnected. Accordingly, any of 0 V and three stages of voltages can be applied to the negative input terminal of the operational amplifier 170 by turning on and off of the switches SW1, SW2, SW3, and SW4. Therefore, an application voltage which decides the heat generating temperature of the heater element 61 can be set in three stages by turning on and off of the switches SW1, SW2, SW3, and SW4.

Here, the temperature of the heater element 61 when a voltage $V_{L1}$ of 1.4 V is applied to the negative input terminal of the operational amplifier 170 is denoted by $T_{H1}$. The temperature of the heater element 61 when a voltage $V_{L2}$ of 1.9 V is applied to the negative input terminal of the operational amplifier 170 is denoted by $T_{H2}$. The temperature of the heater element 61 when a voltage $V_{L3}$ of 2.4 V is applied to the negative input terminal of the operational amplifier 170 is denoted by $T_{H3}$.

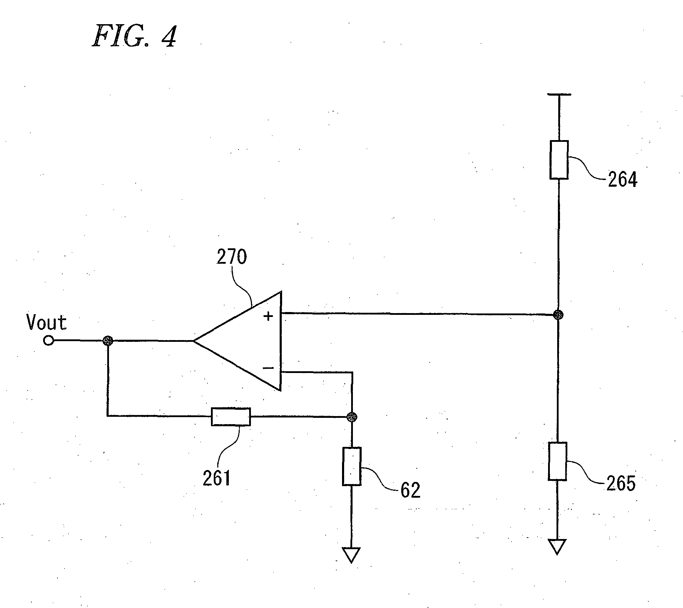

As shown in Fig. 4, the first temperature measurement element 62 is electrically connected to, for example, a negative (-) input terminal of an operational amplifier 270 at one end and is connected to a ground at the other end. In addition, a resistive element 261 is connected in parallel to the negative (-) input terminal and the output terminal of the operational amplifier 270. The positive (+) input terminal of the operational amplifier 270 is electrically connected to somewhere between a resistive element 264 and a resistive element 265 both of which are connected in series. Thereby, a weak voltage of about 0.3 V is applied to the first temperature measurement element 62.

The resistance value of the heater element 61 shown in Figs. 1 and 2 varies depending on the temperature of the heater element 61. The relationship between the temperature $T_H$ of the heater element 61 and the resistance $R_H$ of the heater element 61 is represented by the following Expression (1):

$$R_H = R_{H\_STD} \times [1 + \alpha_H(T_H - T_{H\_STD}) + \beta_H(T_H - T_{H\_STD})^2] \qquad \ldots(1)$$

where the symbol $T_{H\_STD}$ represents the standard temperature of the heater element 61 and is, for example, 20°C. The symbol $R_{H\_STD}$ represents the resistance value of the heater element 61 which is measured at the standard temperature $T_{H\_STD}$ in advance. The symbol $\alpha_H$ represents a primary resistance-temperature coefficient. The symbol $\beta_H$ represents a secondary resistance-temperature coefficient.

The resistance value $R_H$ of the heater element 61 is calculated from the driving power $P_H$ of the heater element 61 and the on-current $I_H$ of the heater element 61 by the following Expression (2):

$$R_H = P_H/I_H{}^2. \qquad \ldots(2)$$

Alternatively, the resistance value $R_H$ of the heater element 61 is calculated from a voltage $V_H$ applied to the heater element 61 and the on-current $I_H$ of the heater element 61 by the following Expression (3):

$$R_H = V_H/I_H. \qquad \ldots(3)$$

Here, the temperature $T_H$ of the heater element 61 is stabilized when the heater element 61 and the atmospheric gas are thermally balanced. The thermally balanced state is a state in which the heat generation of the heater element 61 and the heat loss to the atmospheric gas from the heater element 61 are balanced. As shown in the following Expression (4), by dividing driving power $P_H$ of the heater element 61 in the balanced state by a difference $\Delta T_H$ between the temperature $T_H$ of the heater element 61 and the temperature $T_I$ of the atmospheric gas, a radiation coefficient $M_I$ of the atmospheric gas is obtained. The unit of the radiation coefficient $M_I$ is, for example, W/°C.

$$M_I = P_H/(T_H - T_I)$$
$$= P_H/\Delta T_H \qquad \ldots(4)$$

From the above Expression (1), the temperature $T_H$ of the heater element 61 is represented by the following Expression (5):

$$T_H = (1/2\beta_H) \times [-\alpha_H + [\alpha_H{}^2 - 4\beta_H(1 - R_H/R_{H\_STD})]^{1/2}] + T_{H\_STD} \qquad \ldots(5)$$

Accordingly, the difference $\Delta T_H$ between the temperature $T_H$ of the heater element 61 and the temperature $T_I$ of the atmospheric gas is represented by the following Expression (6):

$$\Delta T_H = (1/2\beta_H) \times [-\alpha_H + [\alpha_H{}^2 - 4\beta_H(1 - R_H/R_{H\_STD})]^{1/2}] + T_{H\_STD} - T_I \qquad \ldots(6)$$

The temperature $T_I$ of the atmospheric gas is close to the temperature $T_I$ of the first temperature measurement element 62 which is supplied with power enough not to generate self-heating. The relationship between the temperature $T_I$ of the first temperature measurement element 62 and the resistance $R_I$ of the first temperature measurement element 62 is represented by the following Expression (7):

$$R_I = R_{I\_STD} \times [1 + \alpha_I(T_I - T_{I\_STD}) + \beta_I(T_I - T_{I\_STD})^2] \qquad \ldots(7)$$

where the symbol $T_{I\_STD}$ represents the standard temperature of the first temperature measurement element 62 and is, for example, 20°C. The symbol $R_{I\_STD}$ represents the resistance value of the first temperature measurement element 62 which is measured at the standard temperature $T_{I\_STD}$ in advance. The symbol $\alpha_I$ represents a primary resistance-temperature coefficient. The symbol $\beta_I$ represents a secondary resistance-temperature coefficient. From the above Expression (7), the temperature $T_I$ of the first temperature measurement element 62 is represented by the following Expression (8):

$$T_I = (1/2\beta_I) \times [-\alpha_I + [\alpha_I{}^2 - 4\beta_I(1 - R_I/R_{I\_STD})]^{1/2}] + T_{I\_STD} \qquad \ldots(8)$$

As a result, the heat loss coefficient $M_I$ of the atmosphere gas is given by the formula (9) below.

$$M_I = P_H/\Delta T_H$$

$$= P_H/[(1/2\beta_H)[-\alpha_H + [\alpha_H{}^2 - 4\beta_H(1 - R_H/R_{H\_STD})]^{1/2}] + T_{H\_STD} - (1/2\beta_I)[-\alpha_I + [\alpha_I{}^2 - 4\beta_I(1 - R_I/R_{I\_STD})^{1/2}] - T_{I\_STD}] \cdots (9)$$

Since the line current $I_H$, driving power $P_H$, or voltage $V_H$ of the heater element 61 can be measured, the resistance value $R_H$ of the heater element 61 can be calculated from the above formula (2) or (3). Likewise, the resistance value $R_I$ of the first temperature measurement element 62 can also be calculated. Therefore, the heat loss coefficient $M_I$ of the atmosphere gas can be calculated from the formula (9) using the microchip 8.

Since the heat-retaining element 64 maintains the temperature of the substrate 60 to be constant, the temperature of the atmosphere gas in the vicinity of the microchip 8 before the heater element 61 generates heat is approximate to the constant temperature of the substrate 60. Therefore, a variation in the temperature of the atmosphere gas before the heater element 61 generates heat is prevented. Since the heater element 61 heats the atmosphere gas whose temperature variation has been prevented, it is possible to calculate the radiation coefficient $M_I$ with high accuracy.

It is assumed that the atmosphere gas is a mixed gas and the mixed gas includes four kinds of gas components A, B, C, and D. The sum of the volume ratio $V_A$ of the gas A, the volume ratio $V_B$ of the gas B, the volume ratio $V_C$ of the gas C, and the volume ratio $V_D$ of the gas D is 1, as represented by the following Expression (10):

$$V_A + V_B + V_C + V_D = 1. \qquad \ldots(10)$$

When the calorific value of the gas A per unit volume is $K_A$, the calorific value of the gas B per unit volume is $K_B$, the calorific value of the gas C per unit volume is $K_C$, and the calorific value of the gas D per unit volume is $K_D$, the calorific value Q of the mixed gas per unit volume is the sum of the values obtained by multiplying the volume ratios of the gas components by the calorific values of the gas components per unit volume. Therefore, the calorific value Q of the mixed gas per unit volume is represented by the following Expression (11):

$$Q = K_A \times V_A + K_B \times V_B + K_C \times V_C + K_D \times V_D. \qquad \ldots(11)$$

The unit of the calorific value per unit volume is, for example, $MJ/m^3$.

When the radiation coefficient of the gas A is $M_A$, the radiation coefficient of the gas B is $M_B$, the radiation coefficient of the gas C is $M_C$, and the radiation coefficient of the gas D is $M_D$, the radiation coefficient $M_I$ of the mixed gas is the sum of the values obtained by multiplying the volume ratios of the gas components by the radiation coefficients of the gas components. Therefore, the radiation coefficient $M_I$ of the mixed gas is represented by the following Expression (12):

$$M_I = M_A \times V_A + M_B \times V_B + M_C \times V_C + M_D \times V_D. \qquad \ldots(12)$$

Further, since the radiation coefficient of gas depends on the heat generating temperature $T_H$ of the heater element 61, the radiation coefficient $M_I$ of the mixed gas is represented as a function of the temperature $T_H$ of the heater element 61 by the following Expression (13):

$$M_I(T_H) = M_A(T_H) \times V_A + M_B(T_H) \times V_B + M_C(T_H) \times V_C + M_D(T_H) \times V_D \qquad \ldots(13)$$

As a result, the heat loss coefficient $M_{I1}(T_{H1})$ of the mixed gas is given by the formula (14) below when the temperature of the heater element 61 is $T_{H1}$. In addition, the heat loss coefficient $M_{I2}(T_{H2})$ of the mixed gas is given by the formula (15) below when the temperature of the heater element 61 is $T_{H2}$, and the heat loss coefficient $M_{I3}(T_{H3})$ of the mixed gas is given by the formula (16) below when the temperature of the heater element 61 is $T_{H3}$.

$$M_{I1}(T_{H1})=M_A(T_{H1})\times V_A+M_B(T_{H1})\times V_B+M_C(T_{H1})\times V_C+M_D(T_{H1})\times V_D \cdots (14)$$

$$M_{I2}(T_{H2})=M_A(T_{H2})\times V_A+M_B(T_{H2})\times V_B+M_C(T_{H2})\times V_C+M_D(T_{H2})\times V_D \cdots (15)$$

$$M_{I3}(T_{H3})=M_A(T_{H3})\times V_A+M_B(T_{H3})\times V_B+M_C(T_{H3})\times V_C+M_D(T_{H3})\times V_D \cdots (16)$$

Here, when the radiation coefficients $M_A(T_H)$, $M_B(T_H)$, $M_C(T_H)$, and $M_D(T_H)$ of the gas components relative to the temperature $T_H$ of the heater element 61 have nonlinearity, the above Expressions (14) to (16) have a linear independent relationship. In addition, even when the radiation coefficients $M_A(T_H)$, $M_B(T_H)$, $M_C(T_H)$, and $M_D(T_H)$ of the gas components relative to the temperature $T_H$ of the heater element 61 have linearity, when the change ratios of the radiation coefficients $M_A(T_H)$, $M_B(T_H)$, $M_C(T_H)$, and $M_D(T_H)$ of the gas components relative to the temperature $T_H$ of the heater element 61 are different from each other, the above Expressions (14) to (16) have a linear independent relationship. Further, when Expressions (14) to (16) have a linear independent relationship, Expressions (10) and (14) to (16) have a linear independent relationship.

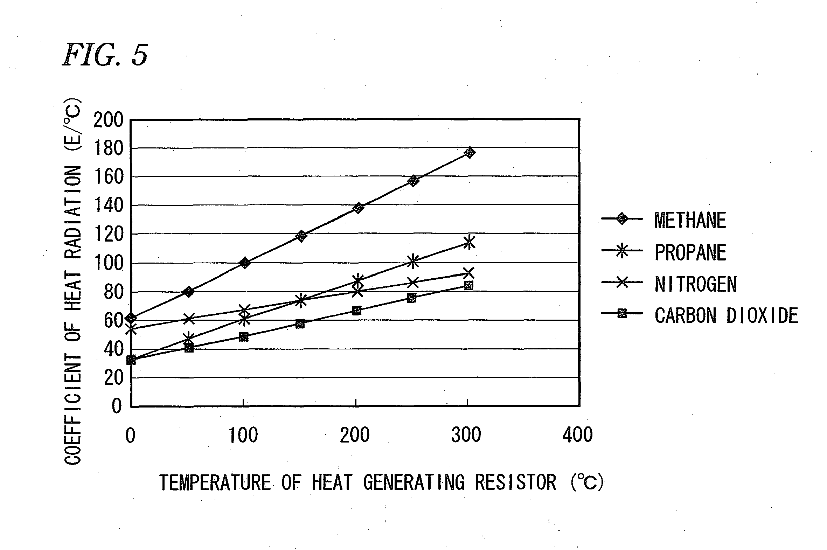

Fig. 5 is a graph illustrating the relationship between the radiation coefficients of methane ($CH_4$), propane ($C_3H_8$), nitrogen ($N_2$), and carbon dioxide ($CO_2$) which are contained in natural gas and the temperature of the heater element 61 which is a heat generating resistor. The radiation coefficients of the gas components of methane ($CH_4$), propane ($C_3H_8$), nitrogen ($N_2$), and carbon dioxide ($CO_2$) relative to the temperature of the heater element 61 have linearity. However, the change ratio of the radiation coefficient relative to the temperature of the heater element 61 is different for each of the cases of methane ($CH_4$), propane ($C_3H_8$), nitrogen ($N_2$), and carbon dioxide ($CO_2$). Therefore, when the gas components of the mixed gas are methane ($CH_4$), propane ($C_3H_8$), nitrogen ($N_2$), and carbon dioxide ($CO_2$) the above Expressions (14) to (16) have a linear independent relationship.

Values of the heat loss coefficients $M_A(T_{H1})$, $M_B(T_{H1})$, $M_C(T_{H1})$, $M_D(T_{H1})$, $M_A(T_{H2})$, $M_B(T_{H2})$, $M_C(T_{H2})$, $M_D(T_{H2})$, $M_A(T_{H3})$, $M_B(T_{H3})$, $M_C(T_{H3})$, and $M_D(T_{H3})$ of the respective gas components in the formula (14) to (16) can be obtained in advance through measurement or the like. Therefore, if solving the simultaneous equations of the formula (10) and (14) to (16), each of the volume ratio $V_A$ of the gas A, the volume ratio $V_B$ of the gas B, the volume ratio $V_C$ of the gas C, and the volume ratio $V_D$ of the gas D is given as a function of the heat loss coefficients $M_{I1}(T_{H1})$, $M_{I2}(T_{H2})$, and $M_{I3}(T_{H3})$ of the mixed gas as shown in the formula (17) to (20) below. Meanwhile, in the formula (17) to (20) below, $f_n$ is a symbol of a function in which n indicates a natural number.

$$V_A=f_1[M_{I1}(T_{H1}), M_{I2}(T_{H2}), M_{I3}(T_{H3})] \cdots (17)$$

$$V_B=f_2[M_{I1}(T_{H1}), M_{I2}(T_{H2}), M_{I3}(T_{H3})] \cdots (18)$$

$$V_C=f_3[M_{I1}(T_{H1}), M_{I2}(T_{H2}), M_{I3}(T_{H3})] \cdots (19)$$

$$V_D=f_4[M_{I1}(T_{H1}), M_{I2}(T_{H2}), M_{I3}(T_{H3})] \cdots (20)$$

Here, the formula (21) below can be obtained by substituting the formula (17) to (20) into the formula (11).

$$Q = K_A \times V_A + K_B \times V_B + K_C \times V_C + K_D \times V_D$$

$$= K_A \times f_1[M_{I1}(T_{H1}), M_{I2}(T_{H2}), M_{I3}(T_{H3})]$$

$$+ K_B \times f_2[M_{I1}(T_{H1}), M_{I2}(T_{H2}), M_{I3}(T_{H3})]$$

$$+ K_C \times f_3[M_{I1}(T_{H1}), M_{I2}(T_{H2}), M_{I3}(T_{H3})]$$

$$+ K_D \times f_4[M_{I1}(T_{H1}), M_{I2}(T_{H2}), M_{I3}(T_{H3})] \cdots (21)$$

As shown in the formula (21), the calorific value Q per unit volume of the mixed gas is given by a formula including the heat loss coefficients $M_{I1}(T_{H1})$, $M_{I2}(T_{H2})$, and $M_{I3}(T_{H3})$ of the mixed gas when the temperatures of the heater element 61 are $T_{H1}$, $T_{H2}$, and $T_{H3}$ as variables. As a result, the calorific value Q of the mixed gas is given by the formula (22) below in which 'g' is a symbol of a function.

$$Q = g[M_{I1}(T_{H1}), M_{I2}(T_{H2}), M_{I3}(T_{H3})] \cdots (22)$$

As a result, the inventors of the invention found that, if the above formula (22) is obtained in advance for the mixed gas made up of the gas A, the gas B, the gas C, and the gas D, the calorific value Q per unit volume of the inspection subject mixed gas of which the volume ratio $V_A$ of the gas A, the volume ratio $V_B$ of the gas B, the volume ratio $V_C$ of the gas C, and the volume ratio $V_D$ of the gas D are unknown can be easily calculated. In detail, by measuring the heat loss coefficients $M_{I1}(T_{H1})$, $M_{I2}(T_{H2})$, and $M_{I3}(T_{H3})$ of the inspection subject mixed gas when the temperatures of the heater element 61 are $T_{H1}$, $T_{H2}$, and $T_{H3}$ and then substituting them into the formula (22), it becomes possible to obtain a unique calorific value Q of the inspection subject mixed gas.

In addition, the heat loss coefficient $M_I$ of the mixed gas is dependent on the resistance value $R_H$ of the heater element 61 and the resistance value $R_I$ of the first temperature measurement element 62 as shown in the formula (9). Therefore, the inventors of the invention found that, as shown in the formula (23) below, the calorific value Q per unit volume of the mixed gas is also given by a formula including the resistance values $R_{H1}(T_{H1})$, $R_{H2}(T_{H2})$, and $R_{H3}(T_{H3})$ of the heater element 61 when the temperatures of the heater element 61 are $T_{H1}$, $T_{H2}$, and $T_{H3}$, and the resistance value $R_I$ of the first temperature measurement element 62 that comes into contact with the mixed gas, as variables.

$$Q = g[R_{H1}(T_{H1}), R_{H2}(T_{H2}), R_{H3}(T_{H3}), R_I] \cdots (23)$$

Therefore, also by measuring the resistance values $R_{H1}(T_{H1})$, $R_{H2}(T_{H2})$, and $R_{H3}(T_{H3})$ of the heater element 61 that comes into the inspection subject mixed gas when the temperatures of the heater element 61 are $T_{H1}$, $T_{H2}$ and $T_{H3}$, and the resistance value $R_I$ of the first temperature measurement element 62 that comes into contact with the inspection subject mixed gas and then substituting them into the formula (23), it becomes possible to obtain a unique calorific value Q of the inspection subject mixed gas.

In addition, as shown in the formula (24) below, the calorific value Q per unit volume of the mixed gas is also given by a formula including the line currents $I_{H1}(T_{H1})$, $I_{H2}(T_{H2})$, and $I_{H3}(T_{H3})$ of the heater element 61 when the temperatures of the heater element 61 are $T_{H1}$, $T_{H2}$, and $T_{H3}$, and the line current $I_I$ of the first temperature measurement element 62 that comes into contact with the mixed gas as variables.

$$Q = g[I_{H1}(T_{H1}), I_{H2}(T_{H2}), I_{H3}(T_{H3}), I_I] \cdots (24)$$

Alternately, as shown in the formula (25) below, the calorific value Q per unit volume of the mixed gas is also given by a formula including the voltages $V_{H1}(T_{H1})$, $V_{H2}(T_{H2})$, and $V_{H3}(T_{H3})$ applied to the heater element 61 when the temperatures of the heater element 61 are $T_{H1}$, $T_{H2}$, and $T_{H3}$, and the voltage $V_I$ applied to the first temperature measurement element 62 that comes into contact with the mixed gas, as variables.

$$Q = g[V_{H1}(T_{H1}), V_{H2}(T_{H2}), V_{H3}(T_{H3}), V_I] \cdots (25)$$

In addition, alternatively, as shown in the formula (26) below, the calorific value Q per unit volume of the mixed gas is also given by a formula including the output signals $AD_{H1}(T_{H1})$, $AD_{H2}(T_{H2})$, and $AD_{H3}(T_{H3})$ of the analog-digital converter (hereinafter referred to as "A/D converter"), which is connected to the heater element 61, when the temperatures of the heater element 61 are $T_{HI}$, $T_{H2}$, and $T_{H3}$, and the output signal $AD_I$ of the A/D converter, which is connected to the first temperature measurement element 62 that comes into contact with the mixed gas, as variables.

$$Q = g[AD_{H1}(T_{H1}), AD_{H2}(T_{H2}), AD_{H3}(T_{H3}), AD_I] \cdots (26)$$

As a result, as shown in the formula (27) below, the calorific value Q per unit volume of the mixed gas is also given by a formula including the electric signals $S_{H1}(T_{H1})$, $S_{H2}(T_{H2})$, and $S_{H3}(T_{H3})$ from the heater element 61 when the temperatures of the heater element 61 are $T_{H1}$, $T_{H2}$, and $T_{H3}$, and the electric signal $S_I$ from the first temperature measurement element 62 that comes into contact with the mixed gas, as variables.

$$Q = g[S_{H1}(T_{H1}), S_{H2}(T_{H2}), S_{H3}(T_{H3}), S_I] \cdots (27)$$

Meanwhile, the gas components of the mixed gas are not limited to 4 kinds. For example, when the mixed gas is made up of n kinds of gas components, firstly, as shown in the formula (28), a formula including the electric signals $S_{H1}(T_{H1})$, $S_{H2}(T_{H2})$, $S_{H3}(T_{H3})$, ... , $S_{Hn-1}(T_{Hn-1})$ from the heater element 61 at the heating temperatures $T_{H1}$, $T_{H2}$, $T_{H3}$, ..., $T_{Hn-1}$ of at least n-1 kinds, and the electric signal $S_I$ from the first temperature measurement element 62 that comes into contact with the mixed gas, which are given in the formula (28) below, as variables, is obtained in advance. Then, the values of the electric signals $S_{H1}(T_{H1})$, $S_{H2}(T_{H2})$, $S_{H3}(T_{H3})$, ..., $S_{Hn-1}(T_{Hn-1})$ from the heater element 61 that comes into contact with the inspection subject mixed gas of which each of the volume ratios of n kinds of gas components are unknown at the heating temperatures $T_{H1}$, $T_{H2}$, $T_{H3}$, ..., $T_{Hn-1}$ of n-1 kinds, and the value of the electric signal $S_I$ from the first temperature measurement element 62 that comes into contact with the inspection subject mixed gas, are measured and substituted into the formula (28), whereby it becomes possible to obtain the unique calorific value Q per unit volume of the inspection subject mixed gas.

$$Q = g[S_{H1}(T_{H1}), S_{H2}(T_{H2}), S_{H3}(T_{H3}), \cdots, S_{Hn-1}(T_{Hn-1}), S_I] \cdots (28)$$

However, if the mixed gas includes alkane ($C_jH_{2j+2}$), in which j is a natural number, other than methane ($CH_4$) and propane ($C_3H_8$) as the gas components in addition to methane ($CH_4$) and propane ($C_3H_8$), the calculation of the formula (28) is not affected even when alkane ($C_jH_{2j+2}$) other than methane ($CH_4$) and propane ($C_3H_8$) is considered as a mixture of methane ($CH_4$) and propane ($C_3H_8$). For example, it makes no change to consider, for example, ethane ($C_2H_6$), butane ($C_4H_{10}$), pentane ($C_5H_{12}$), and hexane ($C_6H_{14}$) as a mixture of methane ($CH_4$) and propane ($C_3H_8$) multiplied by a predetermined coefficient respectively and then calculate the formula (28) as shown in the formula (29) to (32).

$$C_2H_6 = 0.5CH_4 + 0.5C_3H_8 \cdots (29)$$

$$C_4H_{10} = -0.5CH_4 + 1.5C_3H_8 \cdots (30)$$

$$C_5H_{12} = -1.0CH_4 + 2.0C_3H_8 \cdots (31)$$

$$C_6H_{14} = -1.5CH_4 + 2.5C_3H_8 \cdots (32)$$

As a result, when the mixed gas made up of n kinds of gas components includes z kinds of alkane ($C_jH_{2j+2}$) other than methane ($CH_4$) and propane ($C_3H_8$) in addition to methane ($CH_4$) and propane ($C_3H_8$) as the gas components, in which z is a natural number, it is allowed to obtain a formula including the electric signals $S_H$ from the heater element 61 at a minimum of n-z-1 kinds of the heating temperatures and the electric signal $S_I$ from the first temperature measurement element 62 as variables.

When the kind of gas components in the mixed gas used to calculate Expression (28) is equal to the kind of gas components in a mixed gas to be examined whose calorific value Q per unit volume is unknown, Expression (28) may be used to calculate the calorific value Q of the mixed gas to be examined. When the mixed gas to be examined includes gas components that are smaller than n kinds of gas components and gas components smaller than n kinds of gas components are included in the mixed gas used to calculate Expression (28), Expression (28) can be used. For example, when the mixed gas used to calculate Expression (28) includes four kinds of gas components, that is, methane ($CH_4$), propane ($C_3H_8$), nitrogen ($N_2$), and carbon dioxide ($CO_2$) and the mixed gas to be examined does not include nitrogen ($N_2$), but includes only three kinds of gas components, that is, methane ($CH_4$), propane ($C_3H_8$), and carbon dioxide ($CO_2$), Expression (28) can also be used to calculate the calorific value Q of the mixed gas to be examined.

When the mixed gas used to calculate Expression (28) includes as gas components methane ($CH_4$) and propane ($C_3H_8$), Expression (28) can be used even when the mixed gas to be examined includes alkane ($C_jH_{2j+2}$) which is not included in the mixed gas used to calculate Expression (28). This is because regarding alkane ($C_jH_{2j+2}$) other than methane ($CH_4$) and propane ($C_3H_8$) as a mixture of methane ($CH_4$) and propane ($C_3H_8$) does not affect the calculation of the calorific value Q per unit volume by Expression (28), as described above.

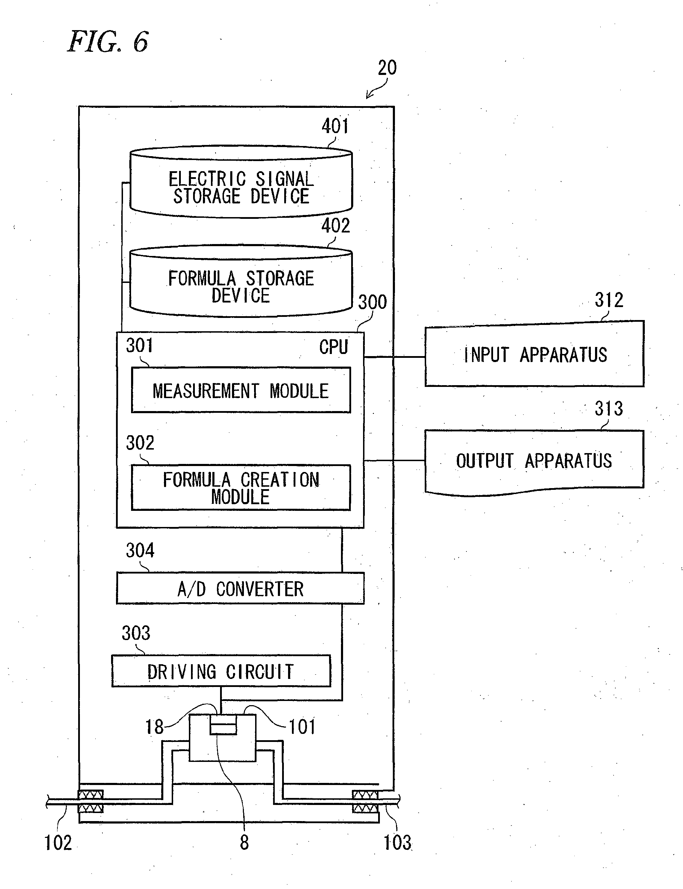

Here, a gas property values measuring system 20 according to the first embodiment shown in Fig. 6 includes a chamber 101, which is a container into which each of a plurality of sample mixed gases is injected, and the microchip 8 which is disposed in the chamber 101 and includes the first temperature measurement element 62 and the heater element 61 that generates heat at a plurality of heating temperatures $T_H$ as shown in Fig. 1. Furthermore, the gas property values measuring system 20 shown in Fig. 6 includes a measurement module 301 that measures the value of the electric signal $S_I$ from the first temperature measurement element 62, which is dependent on the temperature $T_I$ of each of a plurality of sample mixed gases, and the value of the electric signal $S_H$ from the heater element 61 at each of a plurality of heating temperatures $T_H$, and a formula creation module that creates calorific value calculation formula including the electric signal $S_I$ from the first temperature measurement element 62 and the electric signals $S_H$ from the heater element 61 at a plurality of heating temperatures $T_H$ as independent variables and the calorific value Q as a dependant variable based on the values of the known calorific values Q of a plurality of mixed gases, the value of the electric signal $S_I$ from the first temperature measurement element 62, and the values of the electric signals from the heater element 61 at a plurality of heating temperatures. Here, the sample mixed gas includes plural kinds of gas components.

The microchip 8 is arranged in the chamber 101 with the insulating member 18 interposed therebetween. A flow path 102 for transferring the sample mixed gas to the chamber 101 and a flow path 103 of exhausting the sample mixed gas from the chamber 101 to the outside are connected to the chamber 101.

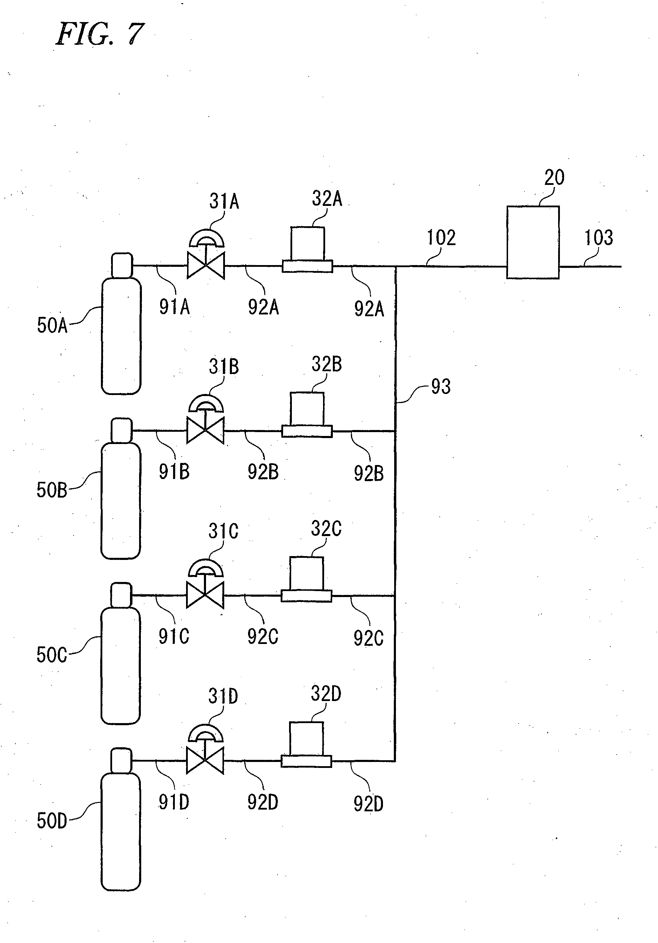

When four kinds of sample mixed gases with different calorific values Q are used, as shown in Fig. 7, a first gas cylinder 50A storing a first sample mixed gas, a second gas cylinder 50B storing a second sample mixed gas, a third gas cylinder 50C storing a third sample mixed gas, and a fourth gas cylinder 50D storing a fourth sample mixed gas are prepared. A first gas pressure adjuster 31A for obtaining the first sample mixed gas which is adjusted to a low pressure of, for example, 0.2 MPa from the first gas cylinder 50A is connected to the first gas cylinder 50A through a flow path 91A. In addition, a first flow rate controller 32A is connected to the first gas pressure adjuster 31A through a flow path 92A. The first flow rate controller 32A controls the flow rate of the first sample mixed gas transferred to the gas property value measurement system 20 through the flow path 92A and a flow path 102.

A second gas pressure adjuster 31B is connected to the second gas cylinder 50B through a flow path 91B. In addition, a second flow rate controller 32B is connected to the second gas pressure adjuster 31B through a flow path 92B. The second flow rate controller 32B controls the flow rate of the second sample mixed gas transferred to the gas property value measurement system 20 tlurough the flow paths 92B, 93, and 102.

A third gas pressure adjuster 31C is connected to the third gas cylinder 50C through a flow path 91C. In addition, a third flow rate controller 32C is collected to the third gas pressure adjuster 31C through a flow path 92C. The third flow rate controller 32C controls the flow rate of the third sample mixed gas transferred to the gas property value measurement system 20 through the flow paths 92C, 93, and 102.

A fourth gas pressure adjuster 31D is connected to the fourth gas cylinder 50D through a flow path 91D. In addition, a fourth flow rate controller 32D is connected to the fourth gas pressure adjuster 31D through a flow path 92D. The fourth flow rate controller 32D controls the flow rate of the fourth sample mixed gas transferred to the gas property value measurement system 20 through the flow paths 92D, 93, and 102.

Each of the first to fourth sample mixed gases is, for example, a natural gas. Each of the first to fourth sample mixed gases includes, for example, four kinds of gas components, that is, methane ($CH_4$), propane ($C_3H_8$), nitrogen ($N_2$), and carbon dioxide ($CO_2$)

After the chamber 10 shown in Fig. 6 is filled with the first sample mixed gas, the first temperature measurement element 62 in the microchip 8 shown in Figs. 1 and 2 outputs electric signals $S_I$ which are dependent on the temperature of the first sample mixed gas. Next, the heater element 61 is supplied with driving power $P_{H1}$, $P_{H2}$, and $P_{H3}$ from a driving circuit 303 shown in Fig. 6. When driving power $P_{H1}$, $P_{H2}$, and $P_{H3}$ is supplied, the heater element 61 that comes into contact with the first sample mixed gas generates heat, for example, at a temperature $T_{H1}$ of 100°C, a temperature $T_{H2}$

of 150°C, a temperature $T_{H3}$ of 200°C, and outputs an electric signal $S_{H1}(T_{H1})$ at the heating temperature $T_{H1}$, an electric signal $S_{H2}(T_{H2})$ at the heating temperature $T_{H2}$, and an electric signal $S_{H3}(T_{H3})$ at the heating temperature $T_{H3}$.

After the first sample mixed gas is removed from the chamber 101, the chamber 101 is sequentially filled with the second to fourth sample mixed gases. After the chamber 101 is filled with the second sample mixed gas, the first temperature measurement element 62 in the microchip 8 shown in Figs. 1 and 2 outputs electric signals $S_I$ which are dependent on the temperature of the second sample mixed gas. Next, the heater element 61 that comes into contact with the second sample mixed gas outputs an electric signal $S_{H1}(T_{H1})$ at the heating temperature $T_{H1}$, an electric signal $S_{H2}(T_{H2})$ at the heating temperature $T_{H2}$, and an electric signal $S_{H3}(T_{H3})$ at the heating temperature $T_{H3}$.

After the chamber 101 shown in Fig. 6 is filled with the third sample mixed gas, the first temperature measurement element 62 in the microchip 8 shown in Figs. 1 and 2 outputs electric signals $S_I$ which are dependent on the temperature of the third sample mixed gas. Next, the heater element 61 that comes into contact with the third sample mixed gas outputs an electric signal $S_{H1}(T_{H1})$ at the heating temperature $T_{H1}$, an electric signal $S_{H2}(T_{H2})$ at the heating temperature $T_{H2}$, and an electric signal $S_{H3}(T_{H3})$ at the heating temperature $T_{H3}$.

After the chamber 101 shown in Fig. 6 is filled with the fourth sample mixed gas, the first temperature measurement element 62 in the microchip 8 shown in Figs. 1 and 2 outputs electric signals $S_I$ which are dependent on the temperature of the fourth sample mixed gas. Next, the heater element 61 that comes into contact with the fourth sample mixed gas outputs an electric signal $S_{H1}(T_{H1})$ at the heating temperature $T_{H1}$, an electric signal $S_{H2}(T_{H2})$ at the heating temperature $TH_2$, and an electric signal $S_{H3}(T_{H3})$ at the heating temperature $T_{H3}$.

Meanwhile, when each of the sample mixed gases includes n kinds of gas components, the heater element 61, shown in Figs. 1 and 2, in the microchip 8 is made to generate heat at a minimum of n-1 kinds of different temperatures. However, as described above, alkane ($C_jH_{2j+2}$) other than methane ($CH_4$) and propane ($C_3H_8$) can be considered as a mixture of methane ($CH_4$) and propane ($C_3H_8$). However, when the sample mixed gas made up of n kinds of gas components includes z kinds of alkane ($C_jH_{2j+2}$) in addition to methane ($CH_4$) and propane ($C_3H_8$) as the gas components, in which z is a natural number, the heater element 61 is made to generate heat at a minimum of n-z-1 kinds of different temperatures.

As shown in Fig. 6, the microchip 8 is connected to a central processing unit (CPU) 300 including the measurement module 301. An electric signals storage device 401 is connected to the CPU 300. The measurement module 301 measures the values of electric signals $S_I$ from the first temperature measurement element 62 and the values of an electric signal $S_{H1}(T_{H1})$ at the heating temperature $T_{H1}$, an electric signal $S_{H2}(T_{H2})$ at the heating temperature $T_{H2}$, and an electric signal $S_{H3}(T_{H3})$ at the heating temperature $T_{H3}$, all of which come from the heater element 61, and saves the measured values in the electric signals storage device 401.

Here, the electric signals $S_I$ from the first temperature measurement element 62 may be any of the resistance value $R_I$ of the first temperature measurement element 62, the line current $I_I$ of the first temperature measurement element 62, the voltage $V_I$ applied to the first temperature measurement element 62, and the output signal $AD_I$ of the A/D converter 304 connected to the heater element 61. Likewise, the electric signals $S_H$ from the heater element 61 may be any of the resistance value $R_H$ of the heater element 61, the line current $I_H$ of the heater element 61, the voltage $V_H$ applied to the heater element 61, and the output signal $AD_H$ of the A/D converter 304 connected to the heater element 61.

The formula creation module 302 included in the CPU 300 collects, for example, the value of the known calorific value Q of each of the first to fourth sample mixed gases, a plurality of measured values of the electric signals $S_I$ from the first temperature measurement element 62, and a plurality of measured values of the electric signals $S_{H1}(T_{H1})$, $S_{H2}(T_{H2})$, and $S_{H3}(T_{H3})$ from the heater element 61. Furthermore, the formula creation module 302 calculates calorific value calculation formula including the electric signals $S_I$ from the first temperature measurement element 62, and the electric signals $S_{H1}(T_{H1})$, $S_{H2}(T_{H2})$, and $S_{H3}(T_{H3})$ from the heater element 61 as independent variables, and the calorific values Q as dependent variables by a multivariate analysis based on the collected values of the calorific values Q, the electric signals $S_I$, and the electric signals $S_H$,

The "multivariate analysis" includes a support vector regression and multiple regression analysis disclosed in A.J. Smola and B. Scholkopf, "A Tutorial on Support Vector Regression" (NeuroCOLT Technical Report (NC-TR-98-030), 1998) and Fuzzy Qualification Theory of Second Order disclosed in JP-A-5-141999.

The gas property values measurement system 20 further includes a formula storage device 402 connected to the CPU 300. The formula storage device 402 saves the calorific value calculation formula built by the formula creation module 302. Furthermore, an input apparatus 312 and an output apparatus 313 are connected to the CPU 300. As the input apparatus 312, for example, a pointing device, such as a keyboard, a mouse, or the like; or the like can be used. As the output apparatus 313, an image display apparatus, such as a liquid crystal display, a monitor, or the like; a printer or the like can be used.

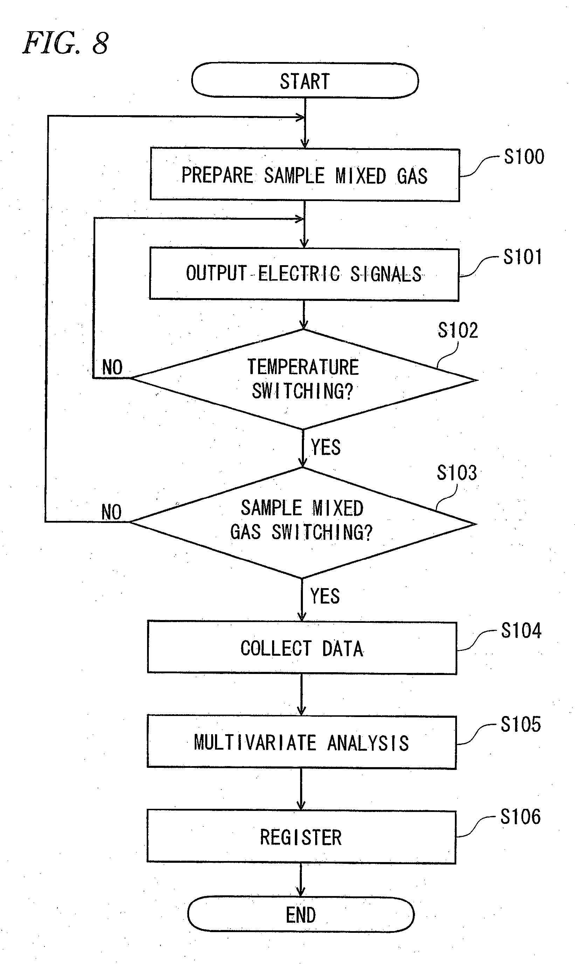

Next, a method of creating a calorific value calculation formula according to the first embodiment will be described using the flowchart shown in Fig. 8.

(a) In Step S100, with the valves of second to fourth flux controllers 32B to 32D shown in Fig. 7 closed, the valve of a first flux controller 32A is opened so that the first sample mixed gas is introduced into the chamber 101 shown in Fig. 6. In Step S101, the measurement module 301 measures the values of the electric signals $S_I$ from the first temperature measurement element 62 that comes into contact with the first sample mixed gas, and saves them in the electric signals storage device 401. Next, the driving circuit 303 supplies a driving power $P_{H1}$ to the heater element 61 shown in Figs. 1 and 2 and makes the heater element 61 generate heat at 100°C. The measurement module 301 shown in Fig. 6 saves the values of the electric signals $S_{H1}(T_{H1})$ from the heater element 61 that generates heat at 100°C in the electric signals storage device 401.

(b) In Step S102, the driving circuit 303 determines whether or not switching of the temperatures of the heater element 61 shown in Figs. 1 and 2 has been completed. When the switching to the temperatures of 150°C and 200°C has not been completed, the operation returns to Step S101, and the driving circuit 303 shown in Fig. 6 makes the heater element 61 shown in Figs. 1 and 2 generate heat at 150°C. The measurement module 301 shown in Fig. 6 saves the values of the electric signals $S_{H2}(T_{H2})$ from the heater element 61 that generates heat at 150°C in the electric signals storage device 401.

(c) Again in Step S102, it is determined whether or not the switching of the temperatures of the heater element 61 shown in Figs. 1 and 2 has been completed. When the switching to the temperature of 200°C has not been completed, the operation returns to Step S101, and the driving circuit 303 shown in Fig. 6 makes the heater element 61 shown in Figs. 1 and 2 generate heat at 200°C. The measurement module 301 shown in Fig. 6 saves the values of the electric signals $S_{H3}(T_{H3})$ from the heater element 61 that generates heat at 200°C in the electric signals storage device 401.

(d) When the switching of the temperatures of the heater element 61 has been completed, the operation proceeds to Step S103 from Step S102. In Step S103, it is determined whether or not switching of the sample mixed gases has been completed. When the switching to the second to fourth sample mixed gases has not been completed, the operation returns to Step S 100. In Step S100, with the valve of the first flux controller 32A closed and the valves of the third to fourth flux controllers 32C to 32D closed, all of which are shown in Fig. 7, the valve of the second flux controller 32B is opened so that the second sample mixed gas is introduced to the chamber 101 shown in Fig. 6.

(e) Similarly to the first sample mixed gas, the loop of Steps S101 to S102 is repeated. The measurement module 301 measures the values of the electric signals $S_I$ from the first temperature measurement element 62 that comes into contact with the second sample mixed gas, and saves them in the electric signals storage device 401. In addition, the measurement module 301 measures the values of the electric signals $S_{H1}(T_{H1})$, $S_{H2}(T_{H2})$, and $S_{H3}(T_{H3})$ from the heater element 61 that comes into contact with the second sample mixed gas and generates heat at 100°C, 150°C, and 200°C, and saves them in the electric signals storage device 401. After that, the loop of Steps S100 to S103 is repeated. Thereby, the values of the electric signals $S_I$ from the first temperature measurement element 62 that comes into contact with the third sample mixed gas, the values of the electric signals $S_{H1}(T_{H1})$, $S_{H2}(T_{H2})$, and $S_{H3}(T_{H3})$ from the heater element 61 that comes into contact with the third sample mixed gas and generates heat at 100°C, 150°C, and 200°C, the values of the electric signals $S_I$ from the first temperature measurement element 62 that comes into contact with the fourth sample mixed gas, and the values of the electric signals $S_{H1}(T_{H1})$, $S_{H2}(T_{H2})$, and $S_{H3}(T_{H3})$ from the heater element 61 that comes into contact with the fourth sample mixed gas and generates heat at 100°C, 150°C, and 200°C are saved in the electric signals storage device 401.

(f) In Step S 104, a value of the known calorific value Q of the first sample mixed gas, a value of the known calorific value Q of the second sample mixed gas, a value of the known calorific value Q of the third sample mixed gas, and a value of the known calorific value Q of the fourth sample mixed gas are provided to the formula creation module 302 from the input apparatus 312. In addition, the formula creation module 302 reads the plurality of measured values of electric signals $S_I$ from the first temperature measurement element 62, and the plurality of measured values of the electric signals $S_{H1}(T_{H1})$, $S_{H2}(T_{H2})$, and $S_{H3}(T_{H3})$ from the heater element 61 from the electric signals storage device 401.

(g) In Step S105, the formula creation module 302 performs a multiple regression analysis based on the values of the calorific values Q of the first to fourth sample mixed gases, the plurality of measured values of electric signals $S_I$ from the first temperature measurement element 62, and the plurality of measured values of the electric signals $S_{H1}(T_{H1})$, $S_{H2}(T_{H2})$, and $S_{H3}(T_{H3})$ from the heater element 61. By the multiple regression analysis, the formula creation module 302 calculates a calorific value calculation formula including the electric signals $S_I$ from the first temperature measurement element 62, and the electric signals $S_{H1}(T_{H1})$, $S_{H2}(T_{H2})$, and $S_{H3}(T_{H3})$ from the heater element 61 as independent variables and the calorific values Q as dependent variables. After that, in Step S106, the formula creation module 302 saves the built calorific value calculation formula in the formula storage device 402 and finishes the method of creating a calorific value calculation formula according to the first embodiment.

As described above, according to the method of creating a calorific value calculation formula according to the first embodiment, it is possible to create a calorific value calculation formula capable of calculating a unique calorific value Q of a measurement target mixed gas.

(Second Embodiment)

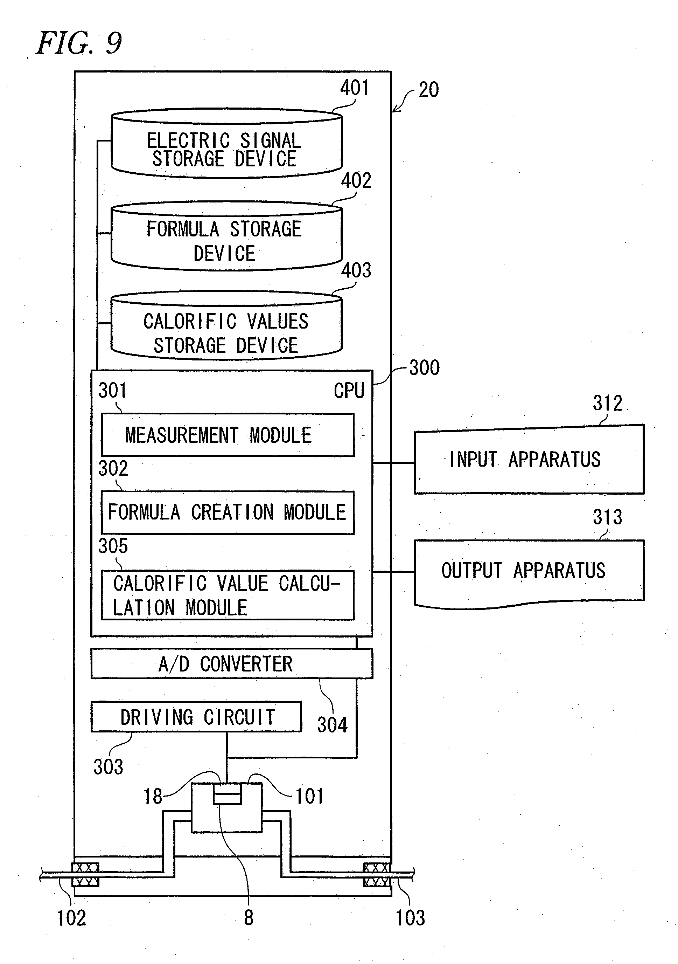

As shown in Fig. 9, a gas property values measurement system 21 according to a second embodiment includes the chamber 101, into which a measurement target mixed gas, with an unknown calorific value Q is injected, and the microchip 8 which is disposed in the chamber 101 and includes the first temperature measurement element 62 and the heater element 61 that generates heat at a plurality of heating temperatures $T_H$ shown in Figs. 1 and 2. The gas property values measuring system 21 shown in Fig. 9 further includes the measurement module 301 that measures the value of the electric signal $S_I$ from the first temperature measurement element 62, which is dependent on the temperature $T_I$ of a measurement target mixed gas, and the value of the electric signal $S_H$ from the heater element 61 at each of a plurality of heating temperatures $T_H$, the formula storage device 402 that saves calorific value calculation formula including the electric signals $S_I$ from the first temperature measurement element 62, and the electric signals $S_H$ from the heater element 61 at a plurality of heating temperatures $T_H$ as independent variables and the calorific values Q as dependent variables, and a calorific value calculation module that substitutes a measured value of the electric signal $S_I$ from the first temperature measurement element 62, and a measured value of the electric signal $S_H$ from the heater element 61 into the independent variable of the electric signal $S_I$ from the first temperature measurement element 62, and the independent variable of the electric signal $S_H$ from the heater element 61 in the calorific value calculation formula so as to calculate a value of the calorific value Q of the measurement target mixed gas.

The formula storage device 402 saves the calorific value calculation formula described in the first embodiment. Here, as an example, a case will be described in which a natural gas including methane ($CH_4$), propane ($C_3H_8$), nitrogen ($N_2$), and carbon dioxide ($CO_2$) is used as a sample mixed gas to create a calorific value calculation formula. In addition, the calorific value calculation formula is considered to include the electric signal $S_I$ from the first temperature measurement element 62, the electric signal $S_{H1}(T_{H1})$ from the heater element 61 at the heating temperature $T_{H1}$ of 100°C, the electric signal $S_{H2}(T_{H2})$ from the heater element 61 at the heating temperature $T_{H2}$ of 150°C, and the electric signal $S_{H3}(T_{H3})$ from the heater element 61 at the heating temperature $T_{H3}$ of 200°C as the independent variables.

In the second embodiment, for example, a natural gas with an unknown calorific value Q including methane ($CH_4$), propane ($C_3H_8$), nitrogen ($N_2$), and carbon dioxide ($CO_2$) in unknown volume ratios is introduced to the chamber 101 as a measurement target mixed gas. The first temperature measurement element 62 in the microchip 8 shown in Figs. 1 and 2 outputs electric signals $S_I$ which are dependent on the temperature of the measurement target mixed gas. Next, the heater element 61 is supplied with driving power $P_{H1}$, $P_{H2}$, and $P_{H3}$ from a driving circuit 303 shown in Fig. 6. When driving power $P_{H1}$, $P_{H2}$, and $P_{H3}$ is supplied, the heater element 61 that comes into contact with the measurement target mixed gas generates heat, for example, at a temperature $T_{H1}$ of 100°C, a temperature $T_{H2}$ of 150°C, a temperature $T_{H3}$ of 200°C, and outputs an electric signal $S_{H1}(T_{H1})$ at the heating temperature $T_{H1}$, an electric signal $S_{H2}(T_{H2})$ at the heating temperature $T_{H2}$, and an electric signal $S_{H3}(T_{H3})$ at the heating temperature $T_{H3}$.

The measurement module 301 shown in Fig. 9 measures the values of the electric signals $S_I$ from the first temperature measurement element 62 that comes into contact with the measurement target mixed gas and the values of an electric signal $S_{H1}(T_{H1})$ at the heating temperature $T_{H1}$, an electric signal $S_{H2}(T_{H2})$ at the heating temperature $T_{H2}$, and an electric signal $S_{H3}(T_{H3})$ at the heating temperature $T_{H3}$, all of which come from the heater element 61 that comes into contact with the measurement target mixed gas, and saves the measured values in the electric signals storage device 401.

The calorific value calculation module 305 substitutes the measured values into the independent variables of the electric signal $S_I$ from the first temperature measurement element 62, and the electric signals $S_{H1}(T_{H1})$, $S_{H2}(T_{H2})$, and $S_{H3}(T_{H3})$ from the heater element 61 in the calorific value calculation formula and calculates the value of a calorific value Q of the measurement target mixed gas. The calorific values storage device 403 is further connected to the CPU 300. The calorific values storage device 403 saves the values of the calorific values Q of the measurement target mixed gases calculated by the calorific value calculation module 305. Since the gas property values measurement system 21 according to the second embodiment has the same conditions as the gas property values measurement system 20 according to the first embodiment described in Fig. 6 with regard to other constitutions, the description thereof will not be repeated.

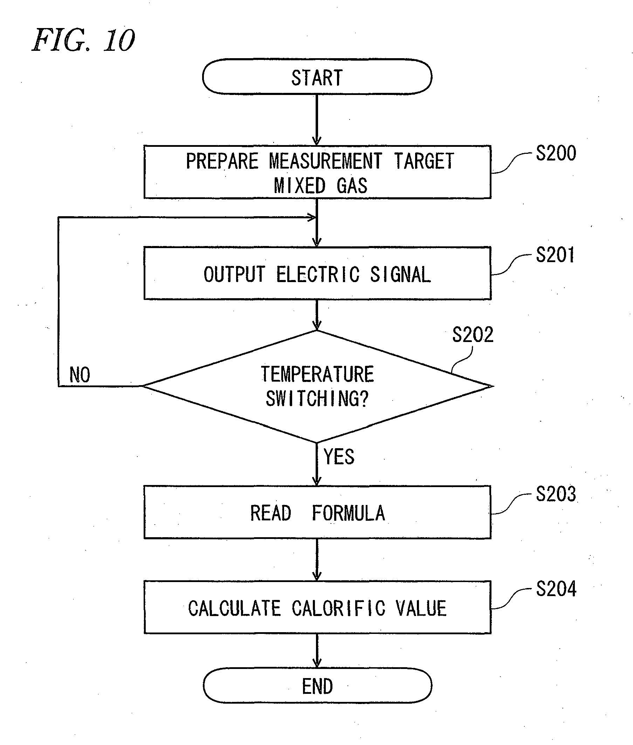

Next, a method of measuring calorific values according to the second embodiment will be described using the flowchart shown in Fig. 10.

(a) In Step S200, the measurement target mixed gas is introduced into the chamber 101 shown in Fig. 9. In Step S201, the measurement module 301 measures the values of the electric signals $S_I$ from the first temperature measurement element 62 that comes into contact with the measurement target mixed gas, and saves them in the electric signals storage device 401. Next, the driving circuit 303 supplies a driving power $P_{H1}$ to the heater element 61 shown in Figs. 1 and 2 and makes the heater element 61 generate heat at 100°C. The measurement module 301 shown in Fig. 9 comes into contact with the measurement target mixed gas and saves the values of the electric signals $S_{H1}(T_{H1})$ from the heater element 61 that generates heat at 100°C in the electric signals storage device 401.

(b) In Step S202, the driving circuit 303 shown in Fig. 9 determines whether or not switching of the temperatures of the heater element 61 shown in Figs. 1 and 2 has been completed. When the switching to the temperatures of 150°C and 200°C has not been completed, the operation returns to Step S201, and the driving circuit 303 supplies driving power $P_{H2}$ to the heater element 61 shown in Figs. 1 and 2, and makes the heater element 61 generate heat at 150°C. The measurement module 301 shown in Fig. 9 comes into contact with the measurement target mixed gas and saves the values of the electric signals $S_{H2}(T_{H2})$ from the heater element 61 that generates heat at 150°C in the electric signals storage device 401.

(c) Again in Step S202, it is determined whether or not the switching of the temperatures of the heater element 61 shown in Figs. 1 and 2 has been completed. When the switching to the temperature of 200°C has not been completed, the operation returns to Step S201, and the driving circuit 303 supplies driving power $P_{H3}$ to the heater element 61 shown in Figs. 1 and 2, and makes the heater element 61 generate heat at 200°C. The measurement module 301 shown in Fig. 9 comes into contact with the measurement target mixed gas and saves the values of the electric signals $S_{H3}(T_{H3})$ from the heater element 61 that generates heat at 200°C in the electric signals storage device 401.

(d) When the switching of the temperatures of the heater element 61 has been completed, the operation proceeds to Step S203 from Step S202. In Step S203, the calorific value calculation module 305 shown in Fig. 9 reads a calorific value calculation formula including the electric signal $S_I$ from the first temperature measurement element 62, and the electric signals $S_{H1}(T_{H1})$, $S_{H2}(TH_2)$, and $S_{H3}(T_{H3})$ from the heater element 61 as independent variables and the calorific values Q as dependent variables from the formula storage device 402. In addition, the calorific value calculation module 305 reads the measured value of the electric signal $S_I$ from the first temperature measurement element 62 that comes into contact with the measurement target mixed gas, and the measured values of the electric signals $S_{H1}(T_{H1})$, $S_{H2}(T_{H2})$, and $S_{H3}(T_{H3})$ from the heater element 61 that comes into contact with the measurement target mixed gas from the electric signals storage device 401.

(e) In Step S204, the calorific value calculation module 305 substitutes each of the measured values into the independent variables of the electric signal $S_I$ and the electric signals $S_{H1}(T_{H1})$, $S_{H2}(T_{H2})$, and $S_{H3}(T_{H3})$ in the calorific value calculation formula, and calculates the value of the calorific value Q of the measurement target mixed gas. After that, the calorific value calculation module 305 saves the value of the calculated calorific value Q in the calorific values storage device 403 and finishes the method of measuring a calorific value according to the second embodiment.

As described above, according to the method of measuring a calorific value according to the second embodiment, it is possible to measure the values of the calorific values Q of mixed gases of measurement target mixed gases from the values of the electric signal $S_I$ from the first temperature measurement element 62 that comes into contact with the measurement target mixed gas, and the values of the electric signals $S_{H1}(T_{H1})$, $S_{H2}(T_{H2})$, and $S_{H3}(T_{H3})$ from the heater element 61 that comes into contact with the measurement target mixed gas without using a high-priced gas chromatography apparatus or sound velocity sensor.

The percentage of carbon hydride in the natural gases varies depending on the gas field. In addition, the natural gases include, for example, nitrogen ($N_2$) or carbon dioxide ($CO_2$) in addition to carbon hydride. Therefore, the volume ratio of gas components in the natural gases varies depending on the gas field. Even when the kind of gas components is known, in many cases, the calorific value Q of the natural gas is unknown. In addition, the natural gases with different calorific values Q may be produced from the same gas field and the calorific value Q of the natural gases varies depending on the production period.

In the related art, when the a price for use of the natural gas is charged, a charging method based on the volume of the natural gas used, not the calorific value Q of the natural gas used has been used. However, it is unfair to charge for the volume of the natural gas used since the calorific value Q of the natural gas varies depending on the gas field of the natural gas. In contrast, when the method of calculating the calorific value according to the second embodiment is used, it is possible to easily calculate the calorific value Q of a mixed gas, such as a natural gas whose calorific value Q is unknown, since the kind of gas components is known, but the volume ratio of the gas components is unknown. Therefore, it is possible to fairly charge for the use of the mixed gas.

In the glass product manufacturing industry, when glass is heated and processed, it is preferable to supply a natural gas with a constant calorific value Q in order to maintain processing accuracy to be constant. In order to maintain processing accuracy, a technique has been examined which accurately checks the calorific values Q of the natural gases produced from a plurality of gas fields, adjusts the calorific values Q of all of the natural gases to be equal to each other, and supplies the natural gas for a process of heating and processing glass. In contrast, when the method of calculating the calorific value according to the second embodiment is used, it is possible to accurately check the calorific values Q of the natural gases produced from a plurality of gas fields. Therefore, it is possible to maintain the heating and processing accuracy of glass to be constant.

According to the method of calculating the calorific value of the fourth embodiment, it is possible to easily know the accurate calorific value Q of a mixed gas, such as a natural gas, and thus appropriately set the amount of air required to burn the mixed gas. Therefore, it is possible to reduce the amount of carbon dioxide ($CO_2$) emitted.

(Example 1)

First, twenty-three kinds of sample mixed gases having known calorific values Q were prepared. Each of the twenty-three kinds of sample mixed gases included as gas components any one or all of methane ($CH_4$), ethane ($C_2H_6$), propane ($C_3H_8$), butane ($C_4H_{10}$), nitrogen ($N_2$), and carbon dioxide ($CO_2$). For example, a sample mixed gas included 90 vol% of methane, 3 vol% of ethane, 1 vol% of propane, 1 vol% of butane, 4 vol% of nitrogen, and 1 vol% of carbon dioxide. A sample mixed gas included 85 vol% of methane, 10 vol% of ethane, 3 vol% of propane, and 2 vol% of butane, but did not include nitrogen and carbon dioxide. A sample mixed gas included 85 vol% of methane, 8 vol% of ethane, 2 vol% of propane, 1 vol% of butane, 2 vol% of nitrogen, and 2 vol% of carbon dioxide.

Next, a plurality of measured values of the electric signals $S_I$ from the first temperature measurement element 62 shown in Fig. 6, and a plurality of measured values of the electric signals $S_{H1}(T_{H1})$, $S_{H2}(T_{H2})$, and $S_{H3}(T_{H3})$ from the heater element 61 were obtained using each of 23 kinds of sample mixed gases. After that, linear equations, quadratic equations and cubic equations were built by a support vector regression based on the values of the known calorific values Q of the 23 kinds of sample mixed gases, the plurality of measured values of the electric signals $S_I$ from the first temperature measurement element 62, and the plurality of measured values of the electric signals $S_{H1}(T_{H1})$, $S_{H2}(T_{H2})$, and $S_{H3}(T_{H3})$ from the heater element 61 to calculate calorific values Q including the electric signals $S_I$ from the first temperature measurement element 62, and the electric signals $S_{H1}(T_{H1})$, $S_{H2}(T_{H2})$, and $S_{H3}(T_{H3})$ from the heater element 61 as independent variables and the calorific values Q as dependent variables.

When creating the linear equations for calculating the calorific values Q, the calibration point can be appropriately determined based on three to five values. The built linear equations are given in the formula (33) to (35) below. The calorific values Q of 23 kinds of the sample mixed gases were calculated using the formula (33) to (35) and compared with the true calorific values Q, which showed the maximum error of 2.1%.

$$Q=40.1+17.4\times V_{H1}(100°C)+17.9\times V_{H2}(150°C)-28.9\times V_{H3}(200°C)-10.4\times V_I \cdots (33)$$

$$Q=40.1+23.8\times R_{H1}(100°C)+6.07\times R_{H2}(150°C)-22.8\times R_{H3}(200°C)-11.4\times R_I \cdots (34)$$

$$Q=40.1+17.4\times AD_{H1}(100°C)+17.9\times AD_{H2}(150°C)-28.9\times AD_{H3}(200°C)-10.4\times AD_I \cdots (35)$$

When creating the quadratic equations for calculating the calorific values Q, the calibration point can be appropriately determined based on eight to nine values. The calorific values Q of 23 kinds of the sample mixed gases were calculated using the built quadratic equations and compared with the true calorific values Q, which showed the maximum error of 1.2% to 1.4%.

When creating the cubic equation for calculating the calorific value Q, it can be appropriately determined that there are approximately ten to fourteen calibration points. When the calorific values Q of the twenty-three kinds of sample mixed gases were calculated by the created cubic equation and compared with true calorific values, the maximum error was less than 1.2%.

(Example 2)

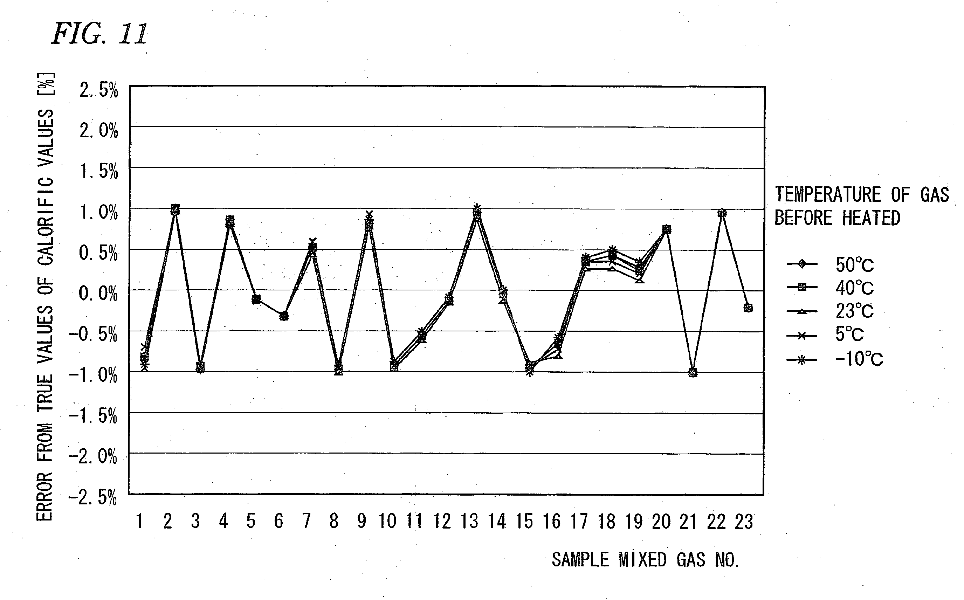

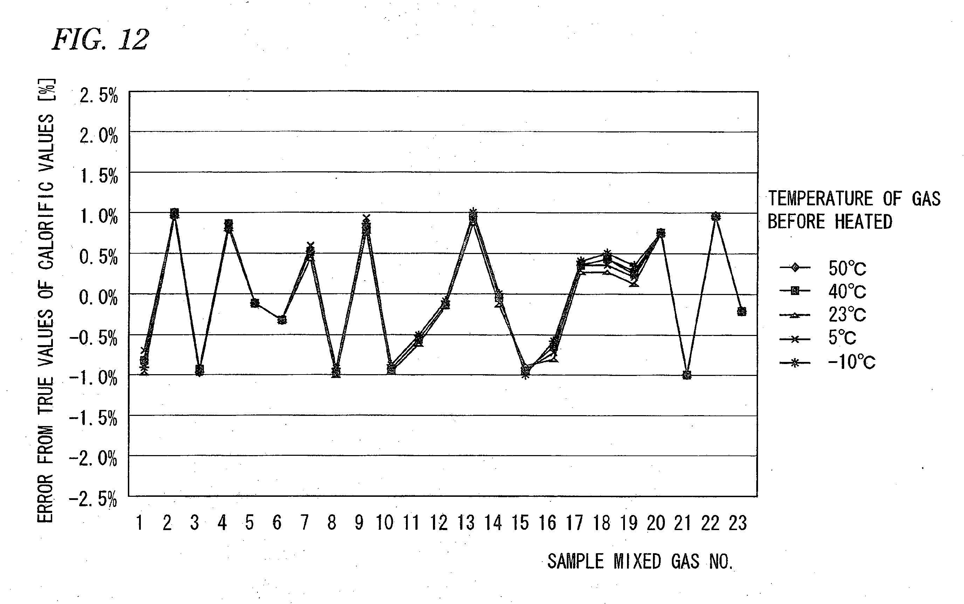

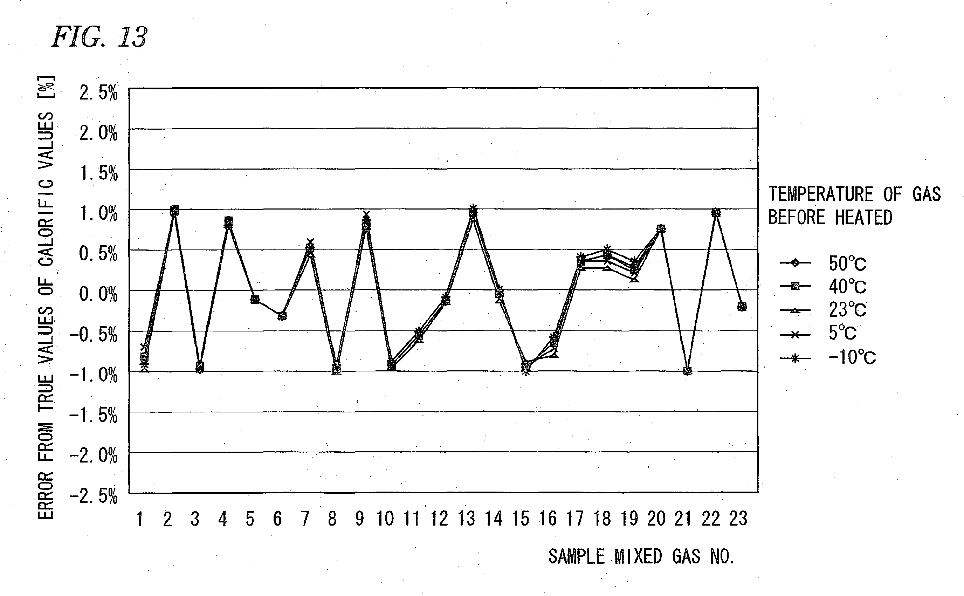

Similarly to the sample mixed gases used in Example 1, 23 kinds of sample mixed gases with known calorific values Q were prepared. Here, the temperature of the sample mixed gases before heated by the heater element 61 were set to -10°C, 5°C, 23°C, 40°C, and 50°C. Next, by a support vector regression, cubic equations for calculating calorific values Q, which includes the electric signals $S_I$ from the first temperature measurement element 62, and the electric signals $S_{H1}(T_{H1})$, $S_{H2}(T_{H2})$, and $S_{H3}(T_{H3})$ from the heater element 61 as independent variables and the calorific values Q as dependent variables, were built. As shown in Figs. 11 to 13, with no regard to the temperatures of the sample mixed gases before heated by the heater element 61, there occurred no variation in the errors of the calculated calorific values Q. Meanwhile, the results in Fig. 11 were obtained using resistances R as the electric signals S. The results in Fig. 12 were obtained using voltages V as the electric signals S. The results in Fig. 13 were obtained using output signals AD from the A/D converter 304 as the electric signals S.

**Claims**

1. A calorific value calculation formula creating system comprising:

   a container (101) into which a gas mixture, including a plurality of gases and having a predetermined calorific value, may be injected;
   a temperature measurement element (62,63) disposed in the container;
   a heater element (61) which is disposed in the container and arranged to generate heat at a plurality of heating temperatures;
   a measurement module (301) arranged to measure first values of electric signals generated from the temperature measurement element and second values of electric signals generated from the heater element at each of the heating temperatures, wherein the first values depend on temperatures of the temperature measurement element at the plurality of gases and the second values depend on temperatures of the heater element; and **characterised by**
   a formula creation module (302) which creates a calorific value calculation formula based on the predetermined calorific values, the first values, and the second values of each of a plurality of gas mixtures,
   wherein the calorific value calculation formula is represented by:

   the first values and the second values, both of which are set as independent variables in the calorific value calculation formula; and
   a calorific value of a gas mixture that is set as a dependent variable in the calorific value calculation formula.

2. The system according to claim **1,** wherein the number of the plurality of gases is known, and the number of the plurality of heating temperatures is at least one less than the number of the plurality of gases.

3. The system according to claim **1** or **2,** wherein the formula creation module creates the calorific value calculation formula using support vector regression.

4. A calorific values measurement system comprising the calorific value calculation formula creation system according to claim **1, 2** or **3,** wherein a gas mixture having an unknown calorific value may be injected into the container (101), and wherein the gas mixture includes a plurality of gases, the system further comprising:

   a formula storage device (402) which stores the calorific value calculation formula; and
   a calorific value calculation module which substitutes the first value and the second values into the first value and the second values which are the independent variables in the calorific value calculation formula, so as to calculate the calorific value of the mixed gas.

5. A method of creating a calorific value calculation formula comprising:

   (a) providing a gas mixture, including a plurality of gases and having a predetermined calorific value, in a container (101);
   (b) measuring first values of electric signals generated from a temperature measurement element (62,63) which is disposed in the container, wherein the first values depend on temperatures of the temperature measurement element at the plurality of gases;
   (c) measuring second values of electric signals generated from a heater element (61) at each of heating temperatures, wherein the heater element is disposed in the container and generates heat at the heating temperatures, and wherein the second values depend on temperatures of the heater element;
   (d) repeating steps (a) to (c) for a plurality of gas mixtures, each including a plurality of gases and having a predetermined calorific value; and **characterised by**
   (e) creating a calorific value calculation formula based on the predetermined calorific values, the first values, and the second values of each of the plurality of gas mixtures,
   wherein the calorific value calculation formula is represented by:

   the first values and the second values, both of which are set as independent variables in the calorific value calculation formula; and

a calorific value of a gas mixture that is set as a dependent variable in the calorific value calculation formula.

6.  The method according to claim **5,**
    wherein the number of the plurality of gases is known, and the number of the plurality of heating temperatures is at least one less than the number of the plurality of gases.

7.  The method according to claim **5** or **6,**
    wherein step (e) comprises creating the calorific value calculation formula using support vector regression.

8.  A method of measuring an unknown calorific value of a gas mixture, the method comprising:

    (a) providing the gas mixture including a plurality of gases in a container (101);
    (b) measuring a first value of an electric signal generated from a temperature measurement element (62,63) which is disposed in the container, wherein the first value depends on a temperature of the temperature measurement element at the gas mixture;
    (c) measuring second values of electrical signals generated from a heater element (61) at each of heating temperatures, wherein the heater element is disposed in the container and generates heat at the heating temperatures;
    (d) reading out a calorific value calculation formula created according to the method of claim **5, 6** or **7,** represented by:

    the first value and the second values, both of which are set as independent variables in the calorific value calculation formula; and
    a calorific value of a gas mixture that is set as a dependent variable in the calorific value calculation formula; and

    (e) substituting the first value and the second values into the first value and the second values which are the independent variables in the calorific value calculation formula, so as to calculate the calorific value of the gas mixture.

**Patentansprüche**

1.  Ein System zur Erzeugung einer Formel zur Brennwertberechnung, das:

    einen Behälter (101), in den eine Gasmischung aus mehreren Gasen und mit einem vorher festgelegten Brennwert eingespritzt werden kann;
    ein Temperaturmessgerät (62, 63), das im Behälter angebracht ist;
    ein Heizelement (61), das im Behälter angebracht und so eingestellt ist, dass es Wärme für verschiedene Heiztemperaturen erzeugen kann;
    ein Messmodul (301), das so eingestellt ist, dass es die ersten Werte elektrischer Signale, die vom Temperaturmessgerät erzeugt werden, und die zweiten Werte elektrischer Signale, die vom Heizelement erzeugt werden, bei jeder Heiztemperatur messen kann, wobei die ersten Werte von den Temperaturen des Temperaturmessgerätes der Vielzahl von Gasen und die zweiten Werte von den Temperaturen des Heizelementes abhängen; und
    **gekennzeichnet durch**
    ein Formelerzeugungsmodul (302), das eine Brennwertberechnungsformel basierend auf den vorher festgelegten Brennwerten, den ersten Werten und den zweiten Werten jeder einzelnen einer Vielzahl von Gasmischungen, erzeugt,
    wobei die Brennwertberechnungsformel **durch**:

    die ersten Werte und die zweiten Werte, die beide als unabhängige Variablen in der Brennwertberechnungsformel angegeben sind; und
    den Brennwert einer Gasmischung, der als abhängige Variable in der Brennwertberechnungsformel angegeben ist, dargestellt wird.

2.  Das System gemäß Anspruch 1, wobei die Anzahl der verschiedenen Gase bekannt ist und die Anzahl der verschiedenen Heiztemperaturen um mindestens eins kleiner ist, als die Anzahl der verschiedenen Gase.

**3.** Das System gemäß Anspruch 1 oder 2, wobei das Formelerzeugungsmodul die Brennwertberechnungsformel unter Nutzung der Support-Vector-Regression erzeugt.

**4.** Ein Brennwertmesssystem, das das System zur Erzeugung einer Formel zur Brennwertberechnung, gemäß Anspruch 1, 2 oder 3 umfasst, wobei eine Gasmischung, die einen unbekannten Brennwert hat, in den Behälter (101) eingespritzt wird und wobei die Gasmischung verschiedene Gase enthält, wobei das System weiterhin:

- eine Vorrichtung zur Formelspeicherung (402), die die Brennwertberechnungsformel speichert; und
- ein Brennwertberechnungsmodul umfasst, das den ersten Wert und die zweiten Werte in den ersten Wert und die zweiten Werte ersetzt, die unabhängige Variablen in der Brennwertberechnungsformel sind, um so den Brennwert der Mischgase zu berechnen.

**5.** Ein Verfahren zur Erzeugung einer Brennwertberechnungsformel, das:

(a) die Lieferung einer Gasmischung, die aus verschiedenen Gasen besteht, mit einem vorher bestimmten Brennwert, in einem Behälter (101);
(b) die Messung erster Werte elektrischer Signale, die von einem Temperaturmessgerät (62, 63) erzeugt werden, das im Behälter montiert ist, wobei die ersten Werte von den Temperaturen des Temperaturmessgerätes bei der Vielzahl von Gasen abhängen;
(c) die Messung zweiter Werte elektrischer Signale, die von einem Heizelement (61) erzeugt werden, bei jeder Heiztemperatur, wobei das Heizelement im Behälter angebracht ist und Wärme zu den Heiztemperaturen erzeugt und wobei die zweiten Werte von den Temperaturen des Heizelementes abhängen;
(d) die Wiederholung der Schritte (a) bis (c) für die verschiedenen Gasmischungen, die jede mehrere Gase enthalten und einen vorher bestimmten Brennwert haben; und **gekennzeichnet durch**
(e) Erzeugung einer Brennwertberechnungsformel basierend auf den vorher bestimmten Brennwerten, den ersten Werten und den zweiten Werten jeder einzelnen einer Vielzahl von Gasmischungen, umfasst, wobei die Brennwertberechnungsformel **durch**:

die ersten Werte und die zweiten Werte, die beide als unabhängige Variablen in der Brennwertberechnungsformel angegeben sind; und
den Brennwert einer Gasmischung, der als abhängige Variable in der Brennwertberechnungsformel angegeben ist, dargestellt wird.

**6.** Das Verfahren gemäß Anspruch 5, wobei die Anzahl der verschiedenen Gase bekannt ist und die Anzahl der verschiedenen Heiztemperaturen wenigstens um eins kleiner ist, als die Anzahl der verschiedenen Gase.

**7.** Das Verfahren gemäß Anspruch 5 oder 6, wobei Schritt (e) die Erzeugung der Brennwertberechnungsformel unter Nutzung der Support-Vector-Regression umfasst.

**8.** Ein Verfahren zur Messung eines unbekannten Brennwertes einer Gasmischung, wobei das Verfahren:

(a) die Lieferung einer Gasmischung, die aus verschiedenen Gasen besteht, in einem Behälter (101)
(b) die Messung eines ersten Wertes eines elektrischen Signals, das von einem Temperaturmessgerät (62, 63) erzeugt wird, das im Behälter montiert ist, wobei der erste Wert von der Temperatur des Temperaturmessgerätes bei der Gasmischung abhängt;
(c) die Messung zweiter Werte elektrischer Signale, die von einem Heizelement (61) erzeugt werden, bei jeder Heiztemperatur, wobei das Heizelement im Behälter angebracht ist und Wärme zu den Heiztemperaturen erzeugt;
(d) das Auslesen einer Brennwertberechnungsformel, die gemäß dem Verfahren gemäß Anspruch 5, 6 oder 7 erzeugt wird, die durch:

den ersten Wert und die zweiten Werte, die beide als unabhängige Variablen in der Brennwertberechnungsformel angegeben sind; und
den Brennwert einer Gasmischung dargestellt wird, der als abhängige Variable in der Brennwertberechnungsformel angegeben ist; und

(e) Ersetzen des ersten Wertes und der zweiten Werte in den ersten Wert und die zweiten Wert umfasst, die unabhängige Variablen in der Brennwertberechnungsformeln sind, um so den Brennwert der Mischgase zu berechnen.

**Revendications**

1. Système de création de formule de calcul de valeur calorifique comprenant :

   un contenant (101) dans lequel un mélange de gaz, comprenant une pluralité de gaz et ayant une valeur calorifique prédéterminée, peut être injecté ;
   un élément de mesure de température (62, 63) disposé dans le contenant ;
   un élément de chauffage (61) qui est disposé dans le contenant et agencé pour générer de la chaleur à une pluralité de températures de chauffage ;
   un module de mesure (301) agencé pour mesurer des premières valeurs de signaux électriques générés par l'élément de mesure de température et des deuxièmes valeurs de signaux électriques générés par l'élément de chauffage à chacune des températures de chauffage, dans lequel les premières valeurs dépendent des températures de l'élément de mesure de température au niveau de la pluralité de gaz et les deuxièmes valeurs dépendent des températures de l'élément de chauffage ; et **caractérisé par**
   un module de création de formule (302) qui crée une formule de calcul de valeur calorifique sur la base des valeurs calorifiques prédéterminées, des premières valeurs, et des deuxièmes valeurs de chacun d'une pluralité de mélanges de gaz,
   dans lequel la formule de calcul de valeur calorifique est représentée par :

   les premières valeurs et les deuxièmes valeurs, les deux étant fixées en tant que variables indépendantes dans la formule de calcul de valeur calorifique ; et
   une valeur calorifique d'un mélange de gaz qui est fixée en tant que variable dépendante dans la formule de calcul de valeur calorifique.

2. Système selon la revendication 1, dans lequel le nombre de la pluralité de gaz est connu, et le nombre de la pluralité de températures de chauffage est inférieur d'au moins un au nombre de la pluralité de gaz.

3. Système selon la revendication 1 ou 2, dans lequel le module de création de formule crée la formule de calcul de valeur calorifique en utilisant une régression par vecteurs de support.

4. Système de mesure de valeurs calorifiques comprenant le système de création de formule de calcul de valeur calorifique selon la revendication 1, 2 ou 3, dans lequel un mélange de gaz ayant une valeur calorifique inconnue peut être injecté dans le contenant (101), et dans lequel le mélange de gaz comprend une pluralité de gaz, le système comprenant en outre :

   un dispositif de mémorisation de formule (402) qui mémorise la formule de calcul de valeur calorifique ; et
   un module de calcul de valeur calorifique qui substitue la première valeur et les deuxièmes valeurs dans la première valeur et les deuxièmes valeurs qui sont les variables indépendantes dans la formule de calcul de valeur calorifique, de manière à calculer la valeur calorifique du mélange de gaz.

5. Procédé de création d'une formule de calcul de valeur calorifique comprenant :

   (a) la fourniture d'un mélange de gaz, comprenant une pluralité de gaz et ayant une valeur calorifique prédéterminée, dans un contenant (101) ;
   (b) la mesure des premières valeurs de signaux électriques générés par un élément de mesure de température (62, 63) qui est disposé dans le contenant, dans lequel les premières valeurs dépendent des températures de l'élément de mesure de température au niveau de la pluralité de gaz ;
   (c) la mesure des deuxièmes valeurs de signaux électriques générés par un élément de chauffage (61) à chacune des températures de chauffage, dans lequel l'élément de chauffage est disposé dans le contenant et génère de la chaleur aux températures de chauffage, et dans lequel les deuxièmes valeurs dépendent des températures de l'élément de chauffage ;
   (d) la répétition des étapes (a) à (c) pour une pluralité de mélanges de gaz, comprenant chacun une pluralité de gaz et ayant une valeur calorifique prédéterminée ; et **caractérisé par**
   (e) la création d'une formule de calcul de valeur calorifique sur la base des valeurs calorifiques prédéterminées, des premières valeurs, et des deuxièmes valeurs de chacun de la pluralité de mélanges de gaz,

dans lequel la formule de calcul de valeur calorifique est représentée par :

les premières valeurs et les deuxièmes valeurs, les deux étant fixées en tant que variables indépendantes dans la formule de calcul de valeur calorifique ; et

une valeur calorifique d'un mélange de gaz qui est fixée en tant que variable dépendante dans la formule de calcul de valeur calorifique.

6. Procédé selon la revendication 5,
dans lequel le nombre de la pluralité de gaz est connu, et le nombre de la pluralité de températures de chauffage est inférieur d'au moins un au nombre de la pluralité de gaz.

7. Procédé selon la revendication 5 ou 6,
dans lequel l'étape (e) comprend la création de la formule de calcul de valeur calorifique en utilisant une régression par vecteurs de support.

8. Procédé de mesure d'une valeur calorifique inconnue d'un mélange de gaz, le procédé comprenant :

(a) la fourniture du mélange de gaz comprenant une pluralité de gaz dans un contenant (101) ;

(b) la mesure d'une première valeur d'un signal électrique généré par un élément de mesure de température (62, 63) qui est disposé dans le contenant, dans lequel la première valeur dépend d'une température de l'élément de mesure de température au niveau du mélange de gaz ;

(c) la mesure de deuxièmes valeurs de signaux électriques générés par un élément de chauffage (61) à chacune des températures de chauffage, dans lequel l'élément de chauffage est disposé dans le contenant et génère de la chaleur aux températures de chauffage ;

(d) la lecture d'une formule de calcul de valeur calorifique créée selon le procédé de la revendication 5, 6 ou 7, représentée par :

la première valeur et les deuxièmes valeurs, les deux étant fixées en tant que variables indépendantes dans la formule de calcul de valeur calorifique ; et

une valeur calorifique d'un mélange de gaz qui est fixée en tant que variable dépendante dans la formule de calcul de valeur calorifique ; et

(e) la substitution de la première valeur et des deuxièmes valeurs dans la première valeur et les deuxièmes valeurs qui sont les variables indépendantes dans la formule de calcul de valeur calorifique, de manière à calculer la valeur calorifique du mélange de gaz.

*FIG. 1*

FIG. 2

## FIG. 3

Vin (5. 0V)

162

Sw1
VL3 (2. 4V)
163

Sw2
VL2 (1. 9V)

170
164

Vout
Sw3
VL1 (1. 4V)
165

+
61
Sw4
VL0 (0V)

161

FIG. 4

*FIG. 5*

EP 2 372 359 B1

*FIG. 6*

20

401

ELECTRIC SIGNAL
STORAGE DEVICE

402

FORMULA STORAGE
DEVICE

300

301                    CPU

MEASUREMENT MODULE

302

FORMULA CREATION
MODULE

312

INPUT APPARATUS

313

OUTPUT APPARATUS

304

A/D CONVERTER

303

DRIVING CIRCUIT

18          101

102          8          103

26

FIG. 7

*FIG. 8*

```
        ┌──────────────┐
        │    START     │
        └──────┬───────┘
               │
               ▼
     ┌───────────────────────┐
     │ PREPARE SAMPLE MIXED GAS │────── S100
     └───────────┬───────────┘
                 │
                 ▼
     ┌───────────────────────┐
     │  OUTPUT ELECTRIC SIGNALS │────── S101
     └───────────┬───────────┘
                 │
                 ▼
          ╱─────────────╲
     NO  ╱  TEMPERATURE   ╲────── S102
    ◄───╲   SWITCHING?    ╱
          ╲─────────────╱
               │ YES
               ▼
          ╱─────────────╲
     NO  ╱ SAMPLE MIXED   ╲────── S103
    ◄───╲ GAS SWITCHING?  ╱
          ╲─────────────╱
               │ YES
               ▼
     ┌───────────────────────┐
     │     COLLECT DATA      │────── S104
     └───────────┬───────────┘
                 │
                 ▼
     ┌───────────────────────┐
     │  MULTIVARIATE ANALYSIS │────── S105
     └───────────┬───────────┘
                 │
                 ▼
     ┌───────────────────────┐
     │      REGISTER         │────── S106
     └───────────┬───────────┘
                 │
                 ▼
        ┌──────────────┐
        │     END      │
        └──────────────┘
```

## FIG. 9

*FIG. 10*

```
          ┌─────────────────┐
          │      START       │
          └─────────────────┘
                   │
                   ▼
    ┌────────────────────────────────┐
    │  PREPARE MEASUREMENT TARGET     │──── S200
    │         MIXED GAS               │
    └────────────────────────────────┘
                   │
                   ▼
    ┌────────────────────────────────┐
    │     OUTPUT ELECTRIC SIGNAL      │──── S201
    └────────────────────────────────┘
                   │
                   ▼
                                    S202
         ◇─────────────────────────◇
    NO   ◇      TEMPERATURE         ◇
◄────────◇      SWITCHING?          ◇
         ◇─────────────────────────◇
                   │ YES
                   ▼
    ┌────────────────────────────────┐
    │        READ   FORMULA           │──── S203
    └────────────────────────────────┘
                   │
                   ▼
    ┌────────────────────────────────┐
    │    CALCULATE CALORIFIC VALUE    │──── S204
    └────────────────────────────────┘
                   │
                   ▼
          ┌─────────────────┐
          │       END        │
          └─────────────────┘
```

*FIG. 11*

EP 2 372 359 B1

FIG. 12

FIG. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004514138 T
- EP 1947450 A

- JP 5141999 A

**Non-patent literature cited in the description**

- **A.J. SMOLA ; B. SCHOLKOPF.** A Tutorial on Support Vector Regression. *NeuroCOLT Technical Report,* 1998